# EUROPÄISCHE PATENTSCHRIFT

(11) **EP 3 880 642 B1**
(45) Veröffentlichungstag und Bekanntmachung des Hinweises auf die Patenterteilung: **14.01.2026**
(21) Anmeldenummer: 19702361.7
(22) Anmeldetag: 23.01.2019
(51) Int. Cl.: C07C 67/03, C07C 67/08, C07C 69/675

(54) **VERFAHREN ZUR HERSTELLUNG VON FETTALKOHOLESTERN VON HYDROXYCARBONSÄUREN**
METHOD FOR PRODUCING FATTY ALCOHOL ESTERS OF HYDROXY CARBOXYLIC ACIDS
PROCÉDÉ DE PRÉPARATION D'ESTERS D'ALCOOL GRAS D'ACIDES HYDROXYCARBOXYLIQUES

(30) Priorität: 17.01.2019 WO PCT/EP2019/051125
(43) Veröffentlichungstag der Anmeldung: 22.09.2021
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/051546
(87) Internationale Veröffentlichungsnummer: WO 2020/147982

(56) Entgegenhaltungen:
- EP-A1- 0 530 866
- WO-A1-2017/213999
- SHARMA, A. ET AL: "Enantio-reversal in Candida rugosa lipase-catalyzed esterification of 3-hydroxybutyric acid", JOURNAL OF MOLECULAR CATALYSIS B: ENZYMATIC , 10(5), 531-534 CODEN: JMCEF8; ISSN: 1381-1177, vol. 10, 22 February 2000 (2000-02-22), pages 531 - 534, XP002794319, DOI: 10.1016/S1381-1177(00)00095-3 10.1016/S1381-1177(00)00095-3

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Fettalkoholestern der 3-Hydroxybuttersäure sowie die auf diese Weise erhältlichen bzw. hergestellten Fettalkoholester der 3-Hydroxybuttersäure und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Ebenfalls betrifft die vorliegende Erfindung ein Verfahren zur Herstellung von Fettalkoholestern der acylierten (z. B. acetylierten) 3-Hydroxybuttersäure (d. h. also mit anderen Worten Fettalkoholestern der 3-Acyloxybuttersäure (z. B. der 3-Acetoxybuttersäure)) sowie die auf diese Weise erhältlichen bzw. hergestellten Fettalkoholester der acylierten 3-Hydroxybuttersäure, d. h. Fettalkoholester der 3-Acyloxybuttersäure, z. B. der 3-Acetoxybuttersäure und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Fettalkoholester der 3-Hydroxybuttersäure und deren acylierte Derivate umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*), *Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Fettalkoholester der 3-Hydroxybuttersäure und deren acylierte Derivate umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Die EP 0 530 866 A1 betrifft Sulphooxyalkanoat-Tenside, bei welchen ein Teil des Tensidmoleküls eine sogenannte "Vorteilsreagenz"-Funktion hat, und Zusammensetzungen, welche diese Tenside enthalten, wobei dieser Vorteilsreagenzanteil vermutlich zum Tragen kommt, wenn das Tensid metabolisiert oder hydrolysiert wird, wobei die verwendeten Tenside gegenüber Calciumionen unempfindlich sein und gut schäumen sollen.

Darüber hinaus betrifft die wissenschaftliche Publikation gemäß SHARMA, A. ET AL: "Enantio-reversal in Candida rugosa lipase-catalyzed esterification of 3-hydroxybutyric acid", Journal of Molecular Catalysis B: Enzymatic, 10(5), 531-534 Coden: JMCEF8; ISSN: 1381-1177, Bd. 10, 22. Februar 2000, Seiten 531-534, XP002794319 die Lipase-(CRL)-katalysierte Veresterung von racemischer 3-Hydroxybuttersäure mit verschiedenen nukleophilen Alkoholen, wobei die Kettenlänge des Alkohols eine wesentliche Rolle in der Enantioselektivität der Reaktion spielt, wobei bei optimalen Bedingungen der (R)-Ester mit einem 95 bis 98 % enantiomeren Überschuss bei der Veresterung mit 1-Hexanol und 1-Octanol in Gegenwart von frisch aktiviertem 4-Å Molsieb erhältlich ist.

Schließlich betrifft die WO 2017/213999 A1 Fettsäure-ß-Hydroxyester-Verbindungen, Fettsäureester von Butandiol und pharmazeutisch verträgliche Salze davon sowie pharmazeutische Zusammensetzungen mit einer oder mehreren Fettsäure-ß-Hydroxyester-Verbindungen und/oder einem oder mehreren Fettsäureestern von Butandiol. Weiterhin betrifft die WO 2017/213999 A1 ein Verfahren zur Behandlung eines Patienten durch Verabreichung eines oder mehrerer Ester an dem Patienten sowie Kits, welche ein oder mehrere der betreffenden Ester enthalten.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Fettalkohole der 3-Hydroxybuttersäure (beta-Hydroxybuttersäure, BHB bzw. 3-BHB) und auch Fettalkohole der acylierten 3-Hydroxybuttersäure (3-Acyloxybuttersäure) jeweils einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Fettalkoholestern der 3-Hydroxybuttersäure (beta-Hydroxybuttersäure, BHB bzw. 3-BHB gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - einen Fettalkoholester der 3-Hydroxybuttersäure (beta-Hydroxybuttersäure, BHB bzw. 3-BHB) gemäß dem diesbezüglichen Anspruch (Anspruch 9) sowie einen Fettalkoholester der acylierten 3-Hydroxybuttersäure (3-Acyloxybuttersäure) gemäß dem diesbezüglichen Anspruch (Anspruch 10).

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 11); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung -einen erfindungsgemäßen Fettalkoholester der 3-Hydroxybuttersäure (beta-Hydroxybuttersäure, BHB bzw. 3-BHB) sowie einen erfindungsgemäßen Fettalkoholester der acylierten 3-Hydroxybuttersäure (3-Acyloxybuttersäure) zur Verwendung bei der prophylaktischen und/oder therapeutischen Behandlung von Erkrankungen des menschlichen oder tierischen Körpers gemäß dem diesbezüglichen unabhängigen Anspruch (Anspruch 13).

Des Weiteren betrifft die vorliegende Erfindung - gemäß einem **fünften** Aspekt der vorliegenden Erfindung - die Verwendung eines erfindungsgemäßen Fettalkoholesters der 3-Hydroxybuttersäure (beta-Hydroxybuttersäure, BHB bzw. 3-BHB) sowie eines erfindungsgemäßen Fettalkoholesters der acylierten 3-Hydroxybuttersäure (3-Acyloxybuttersäure) jeweils zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 14).

Des Weiteren betrifft die vorliegende Erfindung - gemäß einem **sechsten** Aspekt der vorliegenden Erfindung - ein Nahrungsmittel und/oder Lebensmittelerzeugnis gemäß dem diesbezüglichen unabhängigen Anspruch (Anspruch 15).

Schließlich betrifft die vorliegende Erfindung - gemäß einem **siebten** Aspekt der vorliegenden Erfindung - einen erfindungsgemäßen Fettalkoholester der 3-Hydroxybuttersäure (beta-Hydroxybuttersäure, BHB bzw. 3-BHB) sowie einen erfindungsgemäßen Fettalkoholester der acylierten 3-Hydroxybuttersäure (3-Acyloxybuttersäure) jeweils zur Verwendung als Arzneimittel oder Medikament gemäß den diesbezüglichen unabhängigen Ansprüchen (Ansprüchen 16 und 17).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Fettalkoholestern der 3-Hydroxybuttersäure (beta-Hydroxybuttersäure, BHB bzw. 3-BHB),
(A) wobei gemäß einer (ersten) Syntheseroute (A) mindestens eine Verbindung der allgemeinen Formel (la)

   CH₃-CH(OH)-CH₂-C(O)OR¹ (la)

   wobei in der allgemeinen Formel (la) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
   mit mindestens einem Fettalkohol (II), ausgewählt aus C₁₀-C₃₀-Fettalkoholen, insbesondere C₁₀-C₂₄-Fettalkoholen, umgesetzt wird,
   wobei die Umsetzung in Abwesenheit von Lösemitteln und/oder ohne jedwedes Lösemittel durchgeführt wird,
   wobei die Umsetzung in Gegenwart eines Enzyms als Katalysator durchgeführt wird, wobei der Katalysators nach Umsetzung rezykliert wird, und
   wobei bei der Umsetzung gleichzeitig die Verbindung gemäß der allgemeinen Formel (IVa)

      R¹-OH (IVa)
   gebildet wird, wobei in der allgemeinen Formel (IVa) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, wobei die Verbindung gemäß der allgemeinen Formel (IVa) der Umsetzung kontinuierlich entzogen wird;
   oder aber
(B) wobei gemäß einer (zweiten, zu (A) alternativen) Syntheseroute (B) mindestens eine Verbindung der allgemeinen Formel (Ib)

   CH₃-CH(OR²)-CH₂-C(O)-O-C(O)-CH₂-CH(OR²)-CH₃ (Ib)

   wobei in der allgemeinen Formel (Ib) der Rest R² eine Acylgruppe, ausgewählt aus -C(O)-CH₃ (Acetylgruppe) oder - C(O)-C₂H₅ (Propionylgruppe), bevorzugt - C(O) - CH₃ (Acetylgruppe), darstellt,
   mit mindestens einem Fettalkohol (II), ausgewählt aus C₁₀-C₃₀-Fettalkoholen, insbesondere C₁₀-C₂₄-Fettalkoholen, umgesetzt wird, gefolgt von einer Hydrolyse der Acylgruppe;
so dass als Reaktionsprodukt (III) jeweils ein C₁₀-C₃₀-Fettalkoholester der 3-Hydroxybuttersäure, insbesondere ein C₁₀-C₂₄-Fettalkoholester der 3-Hydroxybuttersäure, erhalten wird.

Wie zuvor ausgeführt, hat die Anmelderin nämlich vollkommen überraschend herausgefunden, dass die auf diese Weise hergestellten Fettalkoholester der 3-Hydroxybuttersäure (beta-Hydroxybuttersäure, BHB bzw. 3-BHB) - synonym unter anderem auch als "3-Hydroxybuttersäure-Fettalkoholester" etc. bezeichnet - und darüber hinaus auch die gemäß Syntheseroute (B) vor Hydrolyse der Acylgruppe gebildeten Reaktionszwischenprodukte (d. h. Fettalkoholester der acylierten 3-Hydroxybuttersäure bzw. synonym Fettalkoholester der 3-Acyloxybuttersäure) jeweils effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Fettalkoholester der 3-Hydroxybuttersäure bzw. der acylierten 3-Hydroxybuttersäure, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung von Fettalkoholestern der 3-Hydroxybuttersäure bzw. der acylierten 3-Hydroxybuttersäure mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich Fettalkoholester der 3-Hydroxybuttersäure bzw. der acylierten 3-Hydroxybuttersäure ohne unerwünschte Nebenreaktionen herstellen lassen, insbesondere einstufig.

Das erfindungsgemäße Verfahren ermöglicht somit erstmalig die Bereitstellung untoxischer Fettalkoholester der 3-Hydroxybuttersäure bzw. der acylierten 3-Hydroxybuttersäure aus an sich bekannten, kommerziell verfügbaren und vor allem physiologisch unbedenklichen Komponenten bzw. Edukten (Ausgangsverbindungen). Die resultierenden Fettalkoholester der 3-Hydroxybuttersäure bzw. der acylierten 3-Hydroxybuttersäure können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten Fettalkoholester der 3-Hydroxybuttersäure bzw. der acylierten 3-Hydroxybuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren die Fettalkoholester der 3-Hydroxybuttersäure bzw. der acylierten 3-Hydroxybuttersäure frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, insbesondere durch Enzymkatalyse, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare bzw. einfach zugängliche Edukte und ermöglicht darüber hinaus eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Insbesondere wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das aus dem erfindungsgemäßen Herstellungsverfahren resultierende Rohreaktionsprodukt kann erforderlichenfalls auf einfache Weise und ohne Weiteres, insbesondere mit an sich bekannten Methoden, aufgereinigt werden, insbesondere von gegebenenfalls noch vorhandenen Edukten und/oder gegebenenfalls vorhandenen Nebenprodukten befreit werden (z. B. destillativ und/oder chromatographisch etc.).

### Syntheseroute (A)

Die nachfolgenden Ausführungen beziehen sich auf Syntheseroute (A) des erfindungsgemäßen Verfahrens.

Gemäß einer besonderen Ausführungsform der vorliegenden Erfindung kann gemäß Syntheseroute (A) die Verbindung der allgemeinen Formel (la) entweder in racemischer Form oder aber in Form des (R)-Enantiomers eingesetzt werden. Die (R)-Konfiguration bezieht sich auf das chirale Kohlenstoffatom in 3-Position der Verbindung der allgemeinen Formel (la).

Erfindungsgemäß bevorzugt ist es, wenn gemäß Syntheseroute (A) in der allgemeinen Formel (la) der Rest R¹ Ethyl darstellt.

Mit anderen Worten ist es erfindungsgemäß bevorzugt, dass gemäß Syntheseroute (A) als Verbindung der allgemeinen Formel (la) 3-Hydroxybuttersäureethylester (Ethyl-3-hydroxybutyrat) der Formel CH₃-CH(OH)-CH₂-C(O)OC₂H₅ eingesetzt wird.

Dies ermöglicht eine besonders effiziente Verfahrensführung und hohe Ausbeuten mit minimierter bzw. unterdrückter Nebenproduktbildung. Zudem ist der 3-Hydroxybuttersäureethylester auch in großen Mengen kommerziell verfügbar und zudem ökonomisch effizienter als die freie Säure (d. h. 3-Hydroxybuttersäure) umsetzbar. Insbesondere kann der 3-Hydroxybuttersäureethylester als Ausgangsverbindung großtechnisch z. B. durch Claisen-Kondensation von Ethylacetat gewonnen werden.

Bei dem erfindungsgemäßen Verfahren gemäß Syntheseroute (A) wird die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß der vorliegenden Erfindung wird gemäß Syntheseroute (A) die Umsetzung in Gegenwart eines Enzyms als Katalysator durchgeführt. Dabei wird der Katalysators nach Umsetzung rezykliert.

Wie zuvor ausgeführt, wird gemäß Syntheseroute (A) die Umsetzung in Gegenwart eines Enzyms als Katalysator durchgeführt.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen der vorliegenden Erfindung kann sich gemäß Syntheseroute (A) das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Gemäß einer besonderen Ausführungsform kann gemäß Syntheseroute (A) das Enzym in immobilisierter Form, immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor im Zusammenhang mit der Verwendung eines Katalysators im Allgemeinen dargelegt, wird das Enzym nach der Umsetzung rezykliert.

Gemäß dieser besonderen Ausführungsform des erfindungsgemäßen Verfahrens gemäß Syntheseroute (A) können als zuvor beschriebenes Enzym entsprechende, kommerziell verfügbare Enzyme der vorgenannten Definition verwendet werden (z. B. CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.).

Dabei ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Auch kann die Menge des eingesetzten Enzyms in weiten Bereichen variieren. Insbesondere kann das Enzym in Mengen, bezogen auf die Gesamtmenge der Ausgangsverbindungen (la) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Darüber hinaus kann auch der angewendete Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Was die Menge an Edukten bzw. Ausgangsverbindungen anbelangt, so kann auch diese gemäß Syntheseroute (A) in weiten Bereichen variiert werden.

Unter Berücksichtigung von Verfahrensökonomie und Optimierung des Verfahrensablaufs gemäß Syntheseroute (A), insbesondere im Hinblick auf die Minimierung von Nebenprodukten, ist es vorteilhaft, wenn gemäß Syntheseroute (A) die Verbindung der allgemeinen Formel (la), bezogen auf die Hydroxylgruppen des Fettalkohols (II) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs gemäß Syntheseroute (A), insbesondere im Hinblick auf die Minimierung von Nebenprodukten, ist es vorteilhaft, wenn die Verbindung der allgemeinen Formel (la) und der Fettalkohol (II) in einem Molverhältnis von Verbindung der allgemeinen Formel (la) / Fettalkohol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Gemäß einer bevorzugten Ausführungsform des erfindungsgemäßen Verfahrens gemäß Syntheseroute (A) ist es bevorzugt, dass gemäß Syntheseroute (A) mindestens eine Verbindung der allgemeinen Formel (la')

CH₃-CH(OH)-CH₂-C(O)OC₂H₅ (la')

mit mindestens einem Fettalkohol (II), ausgewählt aus C₁₀-C₃₀-Fettalkoholen, insbesondere C₁₀-C₂₄-Fettalkoholen, umgesetzt wird;
so dass als Reaktionsprodukt (III) ein C₁₀-C₃₀-Fettalkoholester der 3-Hydroxybuttersäure, insbesondere ein C₁₀-C₂₄- Fettalkoholester der 3-Hydroxybuttersäure, erhalten wird.

Im Rahmen des erfindungsgemäßen Verfahrens wird bei der Umsetzung gemäß Syntheseroute (A) gleichzeitig die Verbindung gemäß der allgemeinen Formel (IVa)

R¹-OH (IVa)

gebildet, wobei in der allgemeinen Formel (IVa) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt. Erfindungsgemäß wird gemäß Syntheseroute (A) die Verbindung gemäß der allgemeinen Formel (IVa) der Umsetzung kontinuierlich entzogen, insbesondere mittels vorzugsweise kontinuierlicher destillativer Entfernung.

### Syntheseroute (B)

Die nachfolgenden Ausführungen beziehen sich auf Syntheseroute (B) des erfindungsgemäßen Verfahrens.

Gemäß einer besonderen Ausführungsform der vorliegenden Erfindung kann gemäß Syntheseroute (B) die Verbindung der allgemeinen Formel (Ib) entweder in racemischer Form oder aber in Form des (R)-Enantiomers eingesetzt werden. Die (R)-Konfiguration bezieht sich auf die beiden chiralen Kohlenstoffatome der Verbindung der allgemeinen Formel (Ib), d. h. die nachfolgend mit "* " gekennzeichneten Kohlenstoffatome in der Verbindung der allgemeinen Formel (Ib), wobei diese chiralen Zentren jeweils dem C-Atom in 3-Position der 3-Hydroxybuttersäure entsprechen:

CH₃-C*H(OR²)-CH₂-C(O)-O-C(O)-CH₂-C*H(OR²)-CH₃ (Ib)

Insbesondere ist es im Rahmen des erfindungsgemäßen Verfahrens bevorzugt, wenn gemäß Syntheseroute (B) in der allgemeinen Formel (Ib) der Rest R² eine Gruppe - C(O)-CH₃ (Acetylgruppe) darstellt und/oder wenn gemäß Syntheseroute (B) als Verbindung der allgemeinen Formel (Ib) die Verbindung der Formel CH₃-CH(OAc)-CH₂-C(O)-O-C(O)-CH₂-CH(OAc)-CH₃, wobei der Rest Ac eine Acetylgruppe darstellt, eingesetzt wird.

Insbesondere wird bei dem erfindungsgemäßen Verfahren gemäß Syntheseroute (B) die Umsetzung in Abwesenheit von Lösemitteln und/oder ohne jedwedes Lösemittel durchgeführt. D. h. die Umsetzung gemäß Syntheseroute (B) wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer besonderen Ausführungsform des erfindungsgemäßen Verfahrens gemäß Syntheseroute (B) kann die Umsetzung insbesondere autokatalytisch oder aber alternativ in Gegenwart eines Katalysators, insbesondere einer mineralischen Säure, durchgeführt werden. Vorzugsweise aber wird gemäß Syntheseroute (B) die Umsetzung autokatalytisch durchgeführt.

Sofern die Umsetzung gemäß Syntheseroute (B) in Gegenwart eines Katalysators durchgeführt wird, ist es bevorzugt, wenn gemäß Syntheseroute (B) die Umsetzung in Gegenwart einer mineralischen Säure durchgeführt wird. Insbesondere kann bei dieser Ausführungsform gemäß Syntheseroute (B) der Katalysator und/oder die mineralische Säure ausgewählt werden aus Schwefelsäuren, Halogenwasserstoffsäuren, Phosphorsäuren und deren Mischungen.

Im Rahmen des erfindungsgemäßen Herstellungsverfahrens gemäß Syntheseroute (B) ist es bevorzugt, wenn gemäß Syntheseroute (B) die Umsetzung bei Temperaturen im Bereich von 20 °C bis 150 °C, insbesondere im Bereich von 50 °C bis 140 °C, vorzugsweise im Bereich von 60 °C bis 130 °C, besonders bevorzugt im Bereich von 70 °C bis 125 °C, ganz besonders bevorzugt im Bereich von 75 °C bis 110 °C, durchgeführt wird.

Weiterhin ist es im Rahmen des erfindungsgemäßen Herstellungsverfahrens gemäß Syntheseroute (B) bevorzugt, wenn gemäß Syntheseroute (B) die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

Was die Menge an Edukten bzw. Ausgangsverbindungen anbelangt, so kann auch diese gemäß Syntheseroute (B) in weiten Bereichen variiert werden.

Unter Berücksichtigung von Verfahrensökonomie und Optimierung des Verfahrensablaufs gemäß Syntheseroute (B), insbesondere im Hinblick auf die Minimierung von Nebenprodukten, ist es vorteilhaft, wenn gemäß Syntheseroute (B) die Verbindung der allgemeinen Formel (Ib), bezogen auf die Hydroxylgruppen des Fettalkohols (II), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs gemäß Syntheseroute (B), insbesondere im Hinblick auf die Minimierung von Nebenprodukten, ist es vorteilhaft, wenn gemäß Syntheseroute (B) die Verbindung der allgemeinen Formel (Ib) und der Fettalkohol (II) in einem Molverhältnis von Verbindung der allgemeinen Formel (Ib) / Fettalkohol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Gemäß einer bevorzugten Ausführungsform des erfindungsgemäßen Verfahrens gemäß Syntheseroute (B) ist es bevorzugt, dass gemäß Syntheseroute (B) mindestens eine Verbindung der allgemeinen Formel (Ib')

CH₃-CH(OAc)-CH₂-C(O)-O-C(O)-CH₂-CH(OAc)-CH₃ (Ib')

wobei in der allgemeinen Formel (Ib') der Rest Ac eine Acetylgruppe darstellt,
mit mindestens einem Fettalkohol (II), ausgewählt aus C₁₀-C₃₀-Fettalkoholen, insbesondere C₁₀-C₂₄-Fettalkoholen, umgesetzt wird, gefolgt von einer Hydrolyse der Acylgruppe;
so dass als Reaktionsprodukt (III) ein C₁₀-C₃₀-Fettalkoholester der 3-Hydroxybuttersäure, insbesondere ein C₁₀-C₂₄- Fettalkoholester der 3-Hydroxybuttersäure, erhalten wird.

Im Rahmen des erfindungsgemäßen Verfahrens wird bei der Umsetzung gemäß Syntheseroute (B) gleichzeitig die Verbindung gemäß der allgemeinen Formel (IVb)

CH₃-CH(OR²)-CH₂-C(O)-OH (IVb)

gebildet, wobei in der allgemeinen Formel (IVb) der Rest R² eine Acylgruppe, ausgewählt aus - C(O) - CH₃ (Acetylgruppe) oder - C(O) - C₂H₅ (Propionylgruppe), bevorzugt - C(O)-CH₃ (Acetylgruppe), darstellt. Insbesondere ist es in diesem Zusammenhang bevorzugt, wenn gemäß Syntheseroute (B) die Verbindung gemäß der allgemeinen Formel (IVb) nach erfolgter Umsetzung entfernt wird, insbesondere mittels destillativer Entfernung.

Im Rahmen des erfindungsgemäßen Verfahrens gemäß Syntheseroute (B) schließt sich der Umsetzung der mindestens einen zuvor definierten Verbindung der allgemeinen Formel (Ib) mit dem mindestens einen zuvor definierten Fettalkohol (II) die Hydrolyse der bei dieser Umsetzung gebildeten Acylgruppe an; denn diese Umsetzung führt auch dazu, dass die in 3-Position der 3-Hydroxybuttersäure befindliche Hydroxylfunktion acyliert, insbesondere acetyliert oder propionyliert, wird (d. h. Austausch des Wasserstoffatoms der in 3-Position der 3-Hydroxybuttersäure befindlichen Hydroxylfunktion durch eine Acylgruppe, insbesondere durch eine Acetylgruppe - C(O) - CH₃ oder durch eine Propionylgruppe - C(O) - C₂H₅). Die gemäß Syntheseroute (B) durchgeführte Hydrolyse der Acylgruppe führt dann schließlich dazu, dass das Reaktionsprodukt (III) frei von Acylgruppen ausgebildet ist. Zu diesem Zweck wird erfindungsgemäß gemäß Syntheseroute (B) im Anschluss an die Umsetzung der mindestens einen zuvor definierten Verbindung der allgemeinen Formel (Ib) mit dem mindestens einen zuvor definierten Fettalkohol (II) eine selektive bzw. partielle Hydrolyse der nach Umsetzung in den erhaltenen Umsetzungsprodukten (= Reaktionszwischenprodukten) vorhandenen Acylgruppen durchgeführt. Auf diese Weise lassen sich also Reaktionsprodukte (III) gemäß nachfolgender Definition erhalten, bei welchen im Rahmen der Hydrolyse die Acylgruppe durch ein Wasserstoffatom ersetzt wird und welche folglich eine freie Hydroxylfunktion enthalten (und zwar in der Position des Reaktionsprodukts (III), welche auf die 3-Position des 3-Hydroxybuttersäureteils im Reaktionsprodukt (III) zurückgeht).

Insbesondere ist es bevorzugt, wenn gemäß Syntheseroute (B) die Hydrolyse der Acylgruppe, insbesondere der Acetylgruppe, in Gegenwart eines Katalysators, vorzugsweise eines Enzyms, erfolgt. Dies gewährleistet eine selektive bzw. partielle Hydrolyse der Acylgruppe, insbesondere unter schonenden und ökonomischen Bedingungen, vorzugsweise unter Vermeidung der Bildung von Nebenprodukten.

Insbesondere kann gemäß Syntheseroute (B) das für die Hydrolyse der Acylgruppe, insbesondere der Acetylgruppe, eingesetzte Enzym ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Insbesondere kann sich gemäß Syntheseroute (B) das für die Hydrolyse der Acylgruppe, insbesondere der Acetylgruppe, eingesetzte Enzym ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Insbesondere kann gemäß Syntheseroute (B) das für die Hydrolyse der Acylgruppe, insbesondere der Acetylgruppe, eingesetzte Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Insbesondere kann gemäß Syntheseroute (B) das für die Hydrolyse der Acylgruppe, insbesondere der Acetylgruppe, eingesetzte Enzym in Mengen, bezogen auf die Gesamtmenge der zu hydrolysierenden Verbindung, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden.

Bei dieser besonderen Ausführungsform ist es bevorzugt, wenn gemäß Syntheseroute (B) das für die Hydrolyse der Acylgruppe, insbesondere der Acetylgruppe, eingesetzte Enzym nach der Hydrolyse rezykliert wird.

Die gemäß Syntheseroute (B) durchgeführte Hydrolyse der Acylgruppe, insbesondere der Acetylgruppe, kann insbesondere bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt werden.

Die gemäß Syntheseroute (B) durchgeführte Hydrolyse der Acylgruppe, insbesondere der Acetylgruppe, kann insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Typischerweise wird gemäß Syntheseroute (B) die Hydrolyse der Acylgruppe, insbesondere der Acetylgruppe, in Gegenwart von Wasser durchgeführt.

Gemäß dieser besonderen Ausführungsform des erfindungsgemäßen Verfahrens gemäß Syntheseroute (B) können als zuvor beschriebenes Enzym entsprechende, kommerziell verfügbare Enzyme der vorgenannten Definition verwendet werden (z. B. CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.).

Wie zuvor beschrieben, wird gemäß Syntheseroute (B) die Verbindung der allgemeinen Formel (Ib), wie zuvor definiert, als Edukt eingesetzt. Die Verbindung der allgemeinen Formel (Ib), wie zuvor definiert, ist ohne Weiteres bzw. auf einfache Weise zugänglich.

Was die im Rahmen des erfindungsgemäßen Verfahrens gemäß Syntheseroute (B) eingesetzte Verbindung der allgemeinen Formel (Ib), wie zuvor definiert, anbelangt, so ist diese erhältlich und/oder wird diese erhalten durch Umsetzung von einem Carbonsäureanhydrid der allgemeinen Formel (V)

R²-O-R² (V)

wobei der Rest R² die zuvor angegebene Bedeutung hat, insbesondere von Essigsäureanhydrid (Acetanhydrid) oder Propionsäureanhydrid, vorzugsweise von Essigsäureanhydrid (Acetanhydrid), mit 3-Hydroxybuttersäure.

Insbesondere kann dabei die Umsetzung von Carbonsäureanhydrid der allgemeinen Formel (V) mit 3-Hydroxybuttersäure entsprechend der Reaktionsgleichung 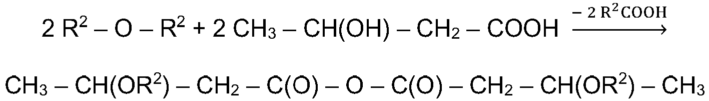 erfolgen, wobei in der Reaktionsgleichung der Rest R² die zuvor angegebene Bedeutung hat.

Gemäß einer besonderen Ausführungsform kann die Umsetzung von Essigsäureanhydrid (Acetanhydrid) mit 3-Hydroxybuttersäure entsprechend der Reaktionsgleichung 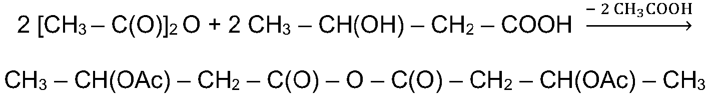 erfolgen, wobei in der Reaktionsgleichung der Rest Ac eine Acetylgruppe darstellt.

Die Temperaturen der Umsetzung von Carbonsäureanhydrid der allgemeinen Formel (V), wie zuvor definiert, mit 3-Hydroxybuttersäure können in weiten Bereichen variieren. Insbesondere kann die Umsetzung von Carbonsäureanhydrid der allgemeinen Formel (V), wie zuvor definiert, mit 3-Hydroxybuttersäure bei Temperaturen im Bereich von 60 bis 150 °C, insbesondere im Bereich von 70 bis 120 °C, vorzugsweise im Bereich von 80 bis 100 °C, durchgeführt werden.

Die Drücke der Umsetzung von Carbonsäureanhydrid der allgemeinen Formel (V), wie zuvor definiert, mit 3-Hydroxybuttersäure können gleichermaßen in weiten Bereichen variieren. Insbesondere kann die Umsetzung von Carbonsäureanhydrid der allgemeinen Formel (V), wie zuvor definiert, mit 3-Hydroxybuttersäure bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer besonderen Ausführungsform des erfindungsgemäßen Verfahrens gemäß Syntheseroute (B) betrifft die vorliegenden Erfindung ein wie zuvor beschriebenes Verfahren zur Herstellung von Fettalkoholestern der 3-Hydroxybuttersäure (beta-Hydroxybuttersäure, BHB bzw. 3-BHB),
wobei gemäß Syntheseroute (B)
   (a) in einem ersten Verfahrensschritt (a) ein Carbonsäureanhydrid der zuvor definierten allgemeinen Formel (V)

      R²-O-R² (V)

      wobei der Rest R² die zuvor angegebene Bedeutung hat, insbesondere Essigsäureanhydrid (Acetanhydrid) oder Propionsäureanhydrid, vorzugsweise Essigsäureanhydrid (Acetanhydrid), mit 3-Hydroxybuttersäure umgesetzt wird, so dass eine Verbindung der allgemeinen Formel (Ib), wie zuvor definiert, erhalten wird; und nachfolgend
   (b) in einem zweiten Verfahrensschritt (b) die auf diese Weise erhaltene Verbindung der allgemeinen Formel (Ib), wie zuvor definiert, mit mindestens einem Fettalkohol (II), ausgewählt aus C₁₀-C₃₀-Fettalkoholen, insbesondere C₁₀-C₂₄-Fettalkoholen, wie zuvor definiert, umgesetzt wird; und nachfolgend
   (c) in einem dritten Verfahrensschritt (c) die Hydrolyse der Acylgruppe erfolgt,
so dass als Reaktionsprodukt (III) ein C₁₀-C₃₀-Fettalkoholester der 3-Hydroxybuttersäure, insbesondere ein C₁₀-C₂₄- Fettalkoholester der 3-Hydroxybuttersäure, erhalten wird.

Wie bereits zuvor im Zusammenhang mit Syntheseroute (B) ausgeführt, führt die gemäß Syntheseroute (B) durchgeführte Umsetzung der mindestens einen zuvor definierten Verbindung der allgemeinen Formel (Ib) mit dem mindestens einen zuvor definierten Fettalkohol (II) auch dazu, dass die in 3-Position der 3-Hydroxybuttersäure befindliche Hydroxylfunktion acyliert, insbesondere acetyliert oder propionyliert, wird (d. h. Austausch des Wasserstoffatoms der in 3-Position der 3-Hydroxybuttersäure befindlichen Hydroxylfunktion durch eine Acylgruppe, insbesondere durch eine Acetylgruppe -C(O)-CH₃ oder durch eine Propionylgruppe - C(O) - C₂H₅). Dieses bei der Umsetzung der mindestens einen zuvor definierten Verbindung der allgemeinen Formel (Ib) mit dem mindestens einen zuvor definierten Fettalkohol (II) erhaltene Umsetzungsprodukt (= Reaktionszwischenprodukt (IIIa)) kann abgetrennt bzw. isoliert werden, ohne dass sich eine Hydrolyse der Acylgruppe anschließt.

Denn, wie bereits zuvor ausgeführt, hat die Anmelderin nämlich vollkommen überraschend herausgefunden, dass auch die gemäß Syntheseroute (B) vor Hydrolyse der Acylgruppe gebildeten Reaktionszwischenprodukte (IIIa) (d. h. Fettalkoholester der acylierten 3-Hydroxybuttersäure bzw. synonym Fettalkoholester der 3-Acyloxybuttersäure) effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind; diese Reaktionszwischenprodukte stellen daher gleichermaßen eine physiologisch und pharmakologisch relevante Alternative zu der freien 3-Hydroxybuttersäure bzw. deren Salzen dar.

Gemäß einer besonderen Ausführungsform gemäß Syntheseroute (B) kann vor der Hydrolyse der Acylgruppe, insbesondere der Acetylgruppe, als Reaktionszwischenprodukt (IIIa) ein C₁₀-C₃₀-Fettalkoholester der acylierten 3-Hydroxybuttersäure (3-Acyloxybuttersäure), insbesondere ein C₁₀-C₂₄-Fettalkoholester der acylierten 3-Hydroxybuttersäure (3-Acyloxybuttersäure), vorzugsweise ein linearer oder verzweigter, gesättigter oder ein- oder mehrfach ungesättigter aliphatischer C₁₀-C₃₀-Fettalkoholester der acylierten 3-Hydroxybuttersäure (3-Acyloxybuttersäure), bevorzugt ein linearer oder verzweigter, gesättigter oder ein- oder mehrfach ungesättigter aliphatischer C₁₀-C₂₄-Fettalkoholester der acylierten 3-Hydroxybuttersäure (3-Acyloxybuttersäure), erhalten werden.

Gemäß einer weiteren besonderen Ausführungsform gemäß Syntheseroute (B) kann vor der Hydrolyse der Acylgruppe, insbesondere der Acetylgruppe, als Reaktionszwischenprodukt (IIIa) ein sich von einem C₁₀-C₃₀-Fettalkohol ableitender Carbonsäureester der acylierten 3-Hydroxybuttersäure (3-Acyloxybuttersäure), insbesondere ein sich von einem C₁₀-C₂₄-Fettalkohol ableitender Carbonsäureester der acylierten 3-Hydroxybuttersäure (3-Acyloxybuttersäure), vorzugsweise ein sich von einem linearen oder verzweigten, gesättigten oder ein- oder mehrfach ungesättigten, aliphatischen einwertigen und bevorzugt primären C₁₀-C₃₀-Fettalkohol ableitender Carbonsäureester der acylierten 3-Hydroxybuttersäure (3-Acyloxybuttersäure), bevorzugt ein sich von einem linearen oder verzweigten, gesättigten oder ein- oder mehrfach ungesättigten, aliphatischen einwertigen und bevorzugt primären C₁₀-C₂₄-Fettalkohol ableitender Carbonsäureester der acylierten 3-Hydroxybuttersäure (3-Acyloxybuttersäure), erhalten werden.

Insbesondere kann in diesem Zusammenhang der C₁₀-C₃₀-Fettalkohol, insbesondere C₁₀-C₂₄-Fettalkohol, ausgewählt sein aus der Gruppe von 1-Decanol, 1-Dodecanol (Laurylalkohol), 1-Tetradecanol (Myristylalkohol), 1-Hexadecanol (Cetylalkohol), 1-Heptadecanol (Margarylalkohol), 1-Octadecanol (Stearylalkohol), 1-Eicosanol (Arachidylalkohol), 1-Docosanol (Behenylalkohol), 1-Tetracosanol (Ligocerylalkohol), 1-Hexacosanol (Cerylalkohol), 1-Octacosanol (Montanylalkohol), 1-Tricontanol (Melissylalkohol), *cis*-9-Hexadecen-1-ol (Palmitoleylalkohol), *cis*-9-Octadecen-1-ol (Oleylalkohol), *trans*-9-Octadecen-1-ol (Elaidylalkohol), *cis*-11-Octadecen-1-ol, *cis,cis*-9,12-Octadecadien-1-ol (Linoleylalkohol), 6,9,12-Octadecatrien-1-ol (γ-Linolenylalkohol), und deren Mischungen, bevorzugt *cis*-9-Octadecen-1-ol (Oleylalkohol).

Erfindungsgemäß bevorzugt ist es in diesem Zusammenhang, wenn die acylierte 3-Hydroxybuttersäure (3-Acyloxybuttersäure) eine acetylierte 3-Hydroxybuttersäure (3-Acetoxybuttersäure) ist.

Gemäß einer wiederum weiteren besonderen Ausführungsform gemäß Syntheseroute (B) kann vor der Hydrolyse der Acylgruppe, insbesondere der Acetylgruppe, als Reaktionszwischenprodukt (IIIa) ein sich von einem linearen, gesättigten oder ein- oder mehrfach ungesättigten aliphatischen, einwertigten primären C₁₀-C₂₄-Fettalkohol ableitender Carbonsäureester der acylierten 3-Hydroxybuttersäure (3-Acyloxybuttersäure), erhalten werden.

Schließlich kann gemäß einer ebenfalls weiteren besonderen Ausführungsform gemäß Syntheseroute (B) vor der Hydrolyse der Acylgruppe, insbesondere der Acetylgruppe, als Reaktionszwischenprodukt (IIIa) ein Fettalkoholester der acylierten 3-Hydroxybuttersäure (3-Acyloxybuttersäure) der allgemeinen Formel (IIIa)

CH₃-CH(OR²)-CH₂-C(O)OR³ (IIIa)

erhalten werden, wobei in der allgemeinen Formel (IIIa)
- R² eine Acylgruppe, ausgewählt aus - C(O) - CH₃ (Acetylgruppe) oder -C(O)-C₂H₅ (Propionylgruppe), bevorzugt - C(O)-CH₃ (Acetylgruppe), darstellt,
- R³ einen linearen oder verzweigten, gesättigten oder ein- oder mehrfach ungesättigten aliphatischen C₁₀-C₃₀-Alkylrest, vorzugsweise C₁₀-C₂₄-Alkylrest, darstellt.

Insbesondere kann dabei in der allgemeinen Formel (IIIa) der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexa-decen-1-ylrest (Palmitoleylrest), einen c*i*s-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octadecadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellen.

### Weiterführende Beschreibung des erfindungsgemäßen Verfahrens im Allgemeinen, insbesondere sowohl gemäß Syntheseroute (A) als auch gemäß Syntheseroute (B)

Alle nun nachfolgenden Ausführungen beziehen sich auf das erfindungsgemäße Verfahren insgesamt bzw. als solches, d. h. sowohl auf Syntheseroute (A) als auch auf Syntheseroute (B) des erfindungsgemäßen Verfahrens.

Sowohl gemäß Syntheseroute (A) als auch gemäß Syntheseroute (B) des erfindungsgemäßen Verfahrens wird jeweils ein Fettalkohol (II), welcher ausgewählt ist aus C₁₀-C₃₀-Fettalkoholen, insbesondere C₁₀-C₂₄-Fettalkoholen, als Edukt eingesetzt.

Gemäß einer besonderen Ausführungsform des erfindungsgemäßen Verfahrens kann es insbesondere vorgesehen sein, wenn der im Rahmen des erfindungsgemäßen Verfahrens einsetzbare Fettalkohol (II) der allgemeinen Formel (II')

R³-OH (II')

entspricht, wobei der Rest R³ einen linearen oder verzweigten, gesättigten oder ein- oder mehrfach ungesättigten aliphatischen C₁₀-C₃₀-Alkylrest, vorzugsweise C₁₀-C₂₄-Alkylrest, darstellt. Insbesondere ist dabei die Hydroxylfunktion (OH-Funktion) primär und/oder endständig.

Insbesondere ist es in diesem Zusammenhang erfindungsgemäß bevorzugt, wenn in der allgemeinen Formel (II') der Rest R³ einen linearen, gesättigten oder ein- oder mehrfach ungesättigten aliphatischen C₁₀-C₂₄-Alkylrest darstellt; insbesondere ist dabei die Hydroxylfunktion (OH-Funktion) primär und/oder endständig.

Insbesondere ist es in diesem Zusammenhang erfindungsgemäß weiterhin bevorzugt, wenn in der allgemeinen Formel (II') der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexadecen-1-ylrest (Palmitoleylrest), einen c*i*s-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octa-decadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellt.

Insbesondere kann der im Rahmen des erfindungsgemäßen Verfahrens einsetzbare Fettalkohol (II) ausgewählt sein aus linearen oder verzweigten, gesättigten oder ein- oder mehrfach ungesättigten aliphatischen C₁₀-C₃₀-Fettalkoholen, insbesondere C₁₀-C₂₄-Fettalkoholen, bevorzugt mit primärer und/oder endständiger Hydroxylfunktion (OH-Funktion).

Gemäß einer besonderen Ausführungsform des erfindungsgemäßen Verfahrens kann der Fettalkohol (II) ausgewählt sein aus ein linearen, gesättigten oder ein- oder mehrfach ungesättigten, aliphatischen einwertigen und bevorzugt primären C₁₀-C₃₀-Fettalkoholen, insbesondere linearen, gesättigten oder ein- oder mehrfach ungesättigten, aliphatischen einwertigen und bevorzugt primären C₁₀-C₂₄-Fettalkoholen.

Gemäß einer weiteren besonderen Ausführungsform des erfindungsgemäßen Verfahrens kann der Fettalkohol (II) ausgewählt sein aus der Gruppe von 1-Decanol, 1-Dodecanol (Laurylalkohol), 1-Tetradecanol (Myristylalkohol), 1-Hexadecanol (Cetylalkohol), 1-Heptadecanol (Margarylalkohol), 1-Octadecanol (Stearylalkohol), 1-Eicosanol (Arachidylalkohol), 1-Docosanol (Behenylalkohol), 1-Tetracosanol (Ligocerylalkohol), 1-Hexacosanol (Cerylalkohol), 1-Octacosanol (Montanylalkohol), 1-Tricontanol (Melissylalkohol), *cis*-9-Hexadecen-1-ol (Palmitoleylalkohol), *cis*-9-Octadecen-1-ol (Oleylalkohol), *trans*-9-Octadecen-1-ol (Elaidylalkohol), *cis*-11-Octadecen-1-ol, *cis,cis*-9,12-Octadecadien-1-ol (Linoleylalkohol), 6,9,12-Octadecatrien-1-ol (γ-Linolenylalkohol), und deren Mischungen, bevorzugt *cis*-9-Octadecen-1-ol (Oleylalkohol).

Die vorgenannten Fettalkohole (II) sind kommerziell verfügbare chemische Erzeugnisse bzw. ohne Weiteres anderweitig zugänglich.

Was die im Rahmen des erfindungsgemäßen Verfahrens erhältlichen Reaktionsprodukte anbelangt, so werden -wie zuvor dargelegt - als Reaktionsprodukt (III) des erfindungsgemäßen Verfahrens (sowohl gemäß Syntheseroute (A) als auch gemäß Syntheseroute (B)) jeweils ein C₁₀-C₃₀-Fettalkoholester der 3-Hydroxybuttersäure, insbesondere ein C₁₀-C₂₄-Fettalkoholester der 3-Hydroxybuttersäure, erhalten.

Insbesondere kann im Rahmen des erfindungsgemäßen Verfahrens als Reaktionsprodukt (III) ein C₁₀-C₃₀-Fettalkoholester der 3-Hydroxybuttersäure, insbesondere ein C₁₀-C₂₄-Fettalkoholester der 3-Hydroxybuttersäure, vorzugsweise ein linearer oder verzweigter, gesättigter oder ein- oder mehrfach ungesättigter aliphatischer C₁₀-C₃₀-Fettalkoholester der 3-Hydroxybuttersäure, bevorzugt ein linearer oder verzweigter, gesättigter oder ein- oder mehrfach ungesättigter aliphatischer C₁₀-C₂₄-Fettalkoholester der 3-Hydroxybuttersäure, erhalten werden.

Insbesondere kann im Rahmen des erfindungsgemäßen Verfahrens als Reaktionsprodukt (III) ein sich von einem C₁₀-C₃₀-Fettalkohol ableitender Carbonsäureester der 3-Hydroxybuttersäure, insbesondere ein sich von einem C₁₀-C₂₄-Fettalkohol ableitender Carbonsäureester der 3-Hydroxybuttersäure, vorzugsweise ein sich von einem linearen oder verzweigten, gesättigten oder ein- oder mehrfach ungesättigten, aliphatischen einwertigen und bevorzugt primären C₁₀-C₃₀-Fettalkohol ableitender Carbonsäureester der 3-Hydroxybuttersäure, bevorzugt ein sich von einem linearen oder verzweigten, gesättigten oder ein- oder mehrfach ungesättigten, aliphatischen einwertigen und bevorzugt primären C₁₀-C₂₄-Fettalkohol ableitender Carbonsäureester der 3-Hydroxybuttersäure, erhalten werden.

Im Rahmen des erfindungsgemäßen Verfahrens kann der C₁₀-C₃₀-Fettalkohol, insbesondere C₁₀-C₂₄-Fettalkohol, des als erfindungsgemäßes Reaktionsprodukt erhaltenen Fettalkoholesters bevorzugt ausgewählt werden aus der Gruppe von 1-Decanol, 1-Dodecanol (Laurylalkohol), 1-Tetradecanol (Myristylalkohol), 1-Hexadecanol (Cetylalkohol), 1-Heptadecanol (Margarylalkohol), 1-Octadecanol (Stearylalkohol), 1-Eicosanol (Arachidylalkohol), 1-Docosanol (Behenylalkohol), 1-Tetracosanol (Ligocerylalkohol), 1-Hexacosanol (Cerylalkohol), 1-Octacosanol (Montanylalkohol), 1-Tricontanol (Melissylalkohol), *cis*-9-Hexadecen-1-ol (Palmitoleylalkohol), *cis*-9-Octadecen-1-ol (Oleylalkohol), *trans*-9-Octadecen-1-ol (Elaidylalkohol), *cis*-11-Octadecen-1-ol, *cis,cis*-9,12-Octadecadien-1-ol (Linoleylalkohol), 6,9,12-Octadecatrien-1-ol (γ-Linolenylalkohol), und deren Mischungen, bevorzugt *cis*-9-Octadecen-1-ol (Oleylalkohol).

Gemäß einer besonderen Ausführungsform des erfindungsgemäßen Verfahrens kann als Reaktionsprodukt (III) ein Fettalkoholester der 3-Hydroxybuttersäure der allgemeinen Formel (III')

CH₃ - CH(OH) - CH₂ - C(O)OR³ (III')

erhalten werden, wobei in der allgemeinen Formel (III') der Rest R³ einen linearen oder verzweigten, gesättigten oder ein- oder mehrfach ungesättigten aliphatischen C₁₀-C₃₀-Alkylrest, vorzugsweise C₁₀-C₂₄-Alkylrest, darstellt.

Gemäß einer weiteren besonderen Ausführungsform des erfindungsgemäßen Verfahrens kann als Reaktionsprodukt (III) ein Fettalkoholester der 3-Hydroxybuttersäure der zuvor angegebenen allgemeinen Formel (III') erhalten werden, wobei in der allgemeinen Formel (III') der Rest R³ einen linearen, gesättigten oder ein- oder mehrfach ungesättigten aliphatischen C₁₀-C₂₄-Alkylrest darstellt.

Gemäß einer weiteren besonderen Ausführungsform des erfindungsgemäßen Verfahrens kann als Reaktionsprodukt (III) ein Fettalkoholester der 3-Hydroxybuttersäure der zuvor angegebenen allgemeinen Formel (III') erhalten werden, wobei in der allgemeinen Formel (III') der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexadecen-1-ylrest (Palmitoleylrest), einen *cis*-9-Octadecen-1-yl (Oleylrest), einen *trans*-9-Octadecen-1-yl (Elaidylrest), einen *cis*-11-Octadecen-1-ylrest, einen *cis,cis*-9,12-Octadecadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellt.

Wie bereits zuvor ausgeführt, hat die Anmelderin vollkommen überraschend herausgefunden, dass die nach dem erfindungsgemäßen Verfahren erhältlichen, zuvor definierten Reaktionsprodukte (d. h. Fettalkoholester der 3-Hydroxybuttersäure) effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind; diese Reaktionsprodukte stellen daher eine physiologisch und pharmakologisch relevante Alternative zu der freien 3-Hydroxybuttersäure bzw. deren Salzen dar.

Im Rahmen des erfindungsgemäßen Herstellungsverfahrens können das Reaktionsprodukt und seine Bildung, insbesondere Umsatz und Ausbeute sowie Selektivität, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Vorsehen eines Katalysators und dessen Auswahl in Bezug auf Art und/oder Menge und/oder Auswahl der Mengen der Ausgangsverbindungen (Edukte) und/oder Vorsehen der Entfernung von Nebenprodukten.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung destillativ und/oder chromatographisch aufgearbeitet bzw. aufgereinigt werden.

Auch können erfindungsgemäß im Anschluss an die Umsetzung bzw. Reaktion gegebenenfalls nichtumgesetzte Ausgangsverbindungen (la) bzw. (Ib) und/oder (II) aus dem Reaktionsprodukt (III) und/oder im Fall der Syntheseroute (B) aus dem Reaktionszwischenprodukt (IIIa) abgetrennt und anschließend rezykliert werden. Diese Vorgehensweise führt zu einer verbesserten Verfahrensökonomie, insbesondere bei großtechnischer bzw. industrieller Durchführung.

Eine erfindungsgemäß bevorzugte Vorgehensweise sowohl gemäß Syntheseroute (A) als auch gemäß Syntheseroute (B) wird durch das nachfolgende Reaktions- bzw. Syntheseschema veranschaulicht (wobei die dort beschriebenen Verbindungen (la), (Ib), (II), (III / III') und (IIIa) jeweils die zuvor angegebene Bedeutung haben, einschließlich der darin verwendeten Reste R¹, R² und R³):

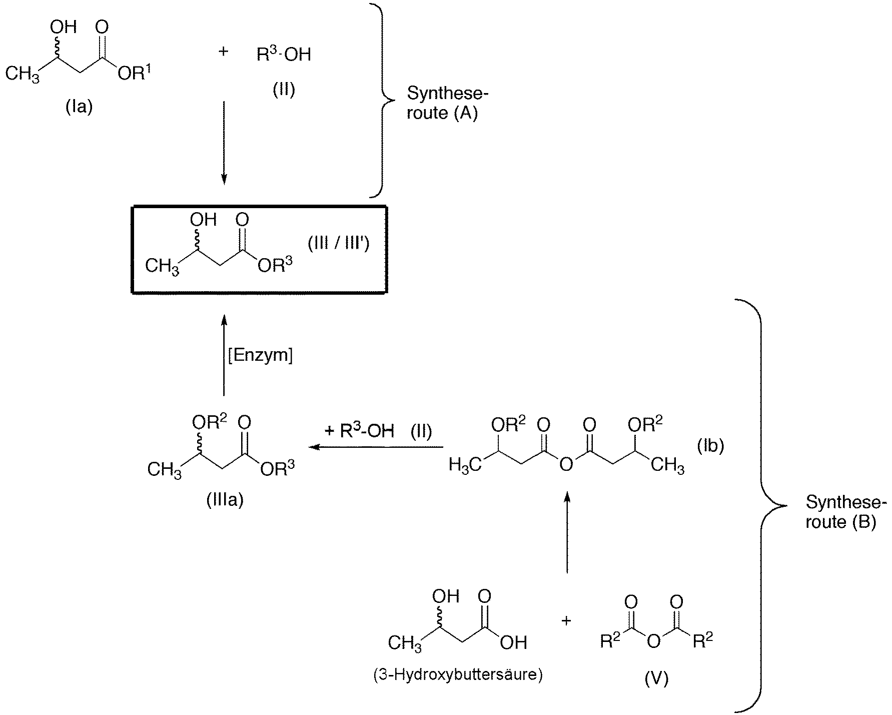

Weiterer Gegenstand - gemäß einem **zweiten** Aspekt der vorliegenden Erfindung - ist ein Fettalkoholester der 3-Hydroxybuttersäure,
wobei der Fettalkoholester der 3-Hydroxybuttersäure der allgemeinen Formel (III')

   CH₃ - CH(OH) - CH₂ - C(O)OR³ (III')
entspricht,
wobei der Rest R³ einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Tricontanylrest (Melissylrest), einen *cis*-9-Hexadecen-1-ylrest (Palmitoleylrest), einen c*i*s-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octa-decadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellt.

Wie zuvor bereits ausgeführt, hat die Anmelderin vollkommen überraschend herausgefunden, dass auch die gemäß Syntheseroute (B) vor Hydrolyse der Acylgruppe gebildeten Reaktionszwischenprodukte (d. h. Fettalkoholester der acylierten 3-Hydroxybuttersäure bzw. synonym auch Fettalkoholester der 3-Acyloxybuttersäure) effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind; diese Reaktionszwischenprodukte stellen daher gleichermaßen eine physiologisch und pharmakologisch relevante Alternative zu der freien 3-Hydroxybuttersäure bzw. deren Salzen dar.

Gegenstand der vorliegenden Erfindung - gemäß dem zweiten Erfindungsaspekt - ist daher auch ein Fettalkoholester der acylierten 3-Hydroxybuttersäure (3-Acyloxybuttersäure),
wobei der Fettalkoholester der acylierten 3-Hydroxybuttersäure (3-Acyloxybuttersäure) ein Fettalkoholester der acylierten 3-Hydroxybuttersäure (3-Acyloxybuttersäure) der allgemeinen Formel (IIIa)

   CH₃ - CH(OR²) - CH₂ - C(O)OR³ (IIIa)

   ist,
wobei in der allgemeinen Formel (IIIa)
   - R² eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
   - der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexadecen-1-ylrest (Palmitoleylrest), einen c*i*s-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octadecadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellt.

Das nach dem erfindungsgemäßen Verfahren erhältliche Reaktionsprodukt, wie zuvor definiert, bzw. der nach dem erfindungsgemäßen Herstellungsverfahren erhältliche bzw. erfindungsgemäße Fettalkoholester der 3-Hydroxybuttersäure, wie zuvor definiert, sowie der Fettalkoholester der acylierten 3-Hydroxybuttersäure (3-Acyloxybuttersäure) (= Reaktionszwischenprodukt vor Hydrolyse gemäß Syntheseroute (B)), wie zuvor definiert, weisen gegenüber dem Stand der Technik eine Vielzahl von Vorteilen und Besonderheiten auf:
Wie die Anmelderin überraschend herausgefunden hat, eignen sich das nach dem erfindungsgemäßen Verfahren erhältliche Reaktionsprodukt, wie zuvor definiert, bzw. der nach dem erfindungsgemäßen Herstellungsverfahren erhältliche bzw. erfindungsgemäße Fettalkoholester der 3-Hydroxybuttersäure, wie zuvor definiert, sowie der Fettalkoholester der acylierten 3-Hydroxybuttersäure (3-Acyloxybuttersäure), wie zuvor definiert, insbesondere als Präkursor bzw. Metabolit von 3-Hydroxybuttersäure bzw. deren Salzen, da diese Verbindungen einerseits physiologisch, insbesondere im Magen/Darm-Trakt, zu 3-Hydroxybuttersäure bzw. deren Salzen umgesetzt werden und andererseits gleichzeitig eine gute physiologische Kompatibilität bzw. Verträglichkeit aufweisen, insbesondere im Hinblick auf Nichttoxizität und akzeptable organoleptische Eigenschaften.

Darüber hinaus sind das nach dem erfindungsgemäßen Verfahren erhältliche Reaktionsprodukt, wie zuvor definiert, bzw. der nach dem erfindungsgemäßen Herstellungsverfahren erhältliche bzw. erfindungsgemäße Fettalkoholester der 3-Hydroxybuttersäure, wie zuvor definiert, sowie der Fettalkoholester der acylierten 3-Hydroxybuttersäure (3-Acyloxybuttersäure), wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem können das nach dem erfindungsgemäßen Verfahren erhältliche Reaktionsprodukt, wie zuvor definiert, bzw. der nach dem erfindungsgemäßen Herstellungsverfahren erhältliche bzw. erfindungsgemäße Fettalkoholester der 3-Hydroxybuttersäure, wie zuvor definiert, sowie der Fettalkoholester der acylierten 3-Hydroxybuttersäure (3-Acyloxybuttersäure), wie zuvor definiert, erforderlichenfalls in enantiomerenreiner bzw. enantiomerenangereicherter Form bereitgestellt werden.

Das nach dem erfindungsgemäßen Verfahren erhältliche Reaktionsprodukt, wie zuvor definiert, bzw. der nach dem erfindungsgemäßen Herstellungsverfahren erhältliche bzw. erfindungsgemäße Fettalkoholester der 3-Hydroxybuttersäure, wie zuvor definiert, sowie der Fettalkoholester der acylierten 3-Hydroxybuttersäure (3-Acyloxybuttersäure), wie zuvor definiert, stellen somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) einen erfindungsgemäßen Fettalkoholester der 3-Hydroxybuttersäure, wie zuvor definiert, und/oder einen erfindungsgemäßen Fettalkoholester der acylierten 3-Hydroxybuttersäure (3-Acyloxybuttersäure) (= Reaktionszwischenprodukt vor Hydrolyse gemäß Syntheseroute (B)), wie zuvor definiert, umfasst.

Insbesondere betrifft die vorliegende Erfindung gemäß diesem Erfindungsaspekt eine pharmazeutische Zusammensetzung zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein erfindungsgemäßer Fettalkoholester der 3-Hydroxybuttersäure, wie zuvor definiert, und/oder ein erfindungsgemäßer Fettalkoholester der acylierten 3-Hydroxybuttersäure (3-Acyloxybuttersäure), wie zuvor definiert, zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **fünften** Aspekt der vorliegenden Erfindung - ist die Verwendung eines erfindungsgemäßen Fettalkoholesters der 3-Hydroxybuttersäure, wie zuvor definiert, und/oder eines Fettalkoholesters der acylierten 3-Hydroxybuttersäure (3-Acyloxybuttersäure), wie zuvor definiert, zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Außerdem kann die Verwendung eines nach dem erfindungsgemäßen Herstellungsverfahren erhältlichen Reaktionsprodukts, wie zuvor definiert, bzw. eines erfindungsgemäßen Fettalkoholesters der 3-Hydroxybuttersäure, wie zuvor definiert, und/oder eines erfindungsgemäßen Fettalkoholesters der acylierten 3-Hydroxybuttersäure (3-Acyloxybuttersäure), wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung, vorgesehen sein.

Gleichermaßen weiterer Gegenstand der vorliegenden Erfindung - gemäß einem **sechsten** Aspekt der vorliegenden Erfindung - ist ein Nahrungsmittel- und/oder Lebensmittelerzeugnis, welches einen erfindungsgemäßen Fettalkoholester der 3-Hydroxybuttersäure, wie zuvor definiert, und/oder einen erfindungsgemäßen Fettalkoholester der acylierten 3-Hydroxybuttersäure (3-Acyloxybuttersäure), wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Auch kann gemäß einer besonderen Ausführungsform der vorliegenden Erfindung die Verwendung eines nach dem erfindungsgemäßen Herstellungsverfahren erhältlichen Reaktionsprodukts, wie zuvor definiert, bzw. eines erfindungsgemäßen Fettalkoholesters der 3-Hydroxybuttersäure, wie zuvor definiert, und/oder eines erfindungsgemäßen Fettalkoholesters der acylierten 3-Hydroxybuttersäure (3-Acyloxybuttersäure), wie zuvor definiert, in einem Nahrungsmittel- und/oder Lebensmittelerzeugnis, vorgesehen sein.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **siebten** Aspekt der vorliegenden Erfindung - ein Fettalkoholester der 3-Hydroxybuttersäure zur Verwendung als Arzneimittel oder Medikament,
wobei der Fettalkoholester der 3-Hydroxybuttersäure der allgemeinen Formel (III')

   CH₃ - CH(OH) - CH₂ - C(O)OR³ (III')
entspricht,
wobei der Rest R³ einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexadecen-1-ylrest (Palmitoleylrest), einen c*i*s-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octa-decadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellt.

Gleichermaßen ist auch Gegenstand der vorliegenden Erfindung gemäß dem siebten Erfindungsaspekt ein Fettalkoholester der acylierten 3-Hydroxybuttersäure (3-Acyloxybuttersäure) zur Verwendung als Arzneimittel oder Medikament,
wobei der Fettalkoholester der acylierten 3-Hydroxybuttersäure (3-Acyloxybuttersäure) ein Fettalkoholester der acylierten 3-Hydroxybuttersäure (3-Acyloxybuttersäure) der allgemeinen Formel (IIIa)

   CH₃ - CH(OR²) - CH₂ - C(O)OR³ (IIIa)

   ist,
wobei in der allgemeinen Formel (IIIa)
   - R² eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
   - der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis*-9-Hexadecen-1-ylrest (Palmitoleylrest), einen c*i*s-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octadecadien-1-ylrest (Linoleylrest) oder einen 6,9,12-Octadecatrien-1-ylrest (γ-Linolenylrest), bevorzugt einen c*i*s-9-Octadecen-1-ylrest (Oleylrest), darstellt.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

- 3-BHB-Ethyl = 3-Hydroxybuttersäureethylester
- 3-BHB-Decyl = 3-Hydroxybuttersäuredecylester
- 3-BHB-Oleyl = 3-Hydroxybuttersäureoleylester
- Dimer(e) = Dimer(e) der 3-Hydroxybuttersäure und/oder des 3-Hydroxybuttersäureethylesters (Reaktionsnebenprodukte)

### Herstellungsbeispiele

Das erfindungsgemäße Herstellungsverfahren wird anhand der nachfolgenden Ausführungsbeispiele veranschaulicht. Das diesbezügliche allgemeine Reaktionsschema hierzu ist im allgemeinen Beschreibungsteil dargestellt und erläutert.

### Herstellung von 3-BHB-Decylester gemäß Syntheseroute (A)

In einem 500-ml-Mehrhalskolben mit Dephlegmator (Partialkondensator) und Destillationsbrücke werden 132 g (R)/(S)-3-Hydroxybuttersäureethylester, 158 g 1-Decanol und 2,9 g immobilisiertem Enzym (CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.) vorgelegt.

Das Reaktionsgemisch wird unter Rühren bei 70°C und unter Vakuum (< 500 mbar) für 7 h zur Reaktion gebracht. Anschließend wird das Enzym abfiltriert und der überschüssige 3-Hydroxybuttersäureethylester bzw. das überschüssige 1-Decanol unter Vakuum abdestilliert. Der erhaltene Rückstand wird für 2 bis 4 h im Hochvakuum gedämpft (Dampftemperatur 160 °C). Es wird reiner 3-BHB-Decylester erhalten.

### Herstellung von 3-BHB-Oleylester gemäß Syntheseroute (A)

In einem 500-ml-Mehrhalskolben mit Dephlegmator (Partialkondensator) und Destillationsbrücke werden 132 g (R)/(S)-3-Hydroxybuttersäureethylester, 270 g Oleylalkohol (Reinheit: 85%) und 4,0 g immobilisiertem Enzym (CALB-Lipase auf Polymerträger, abgeleitet von *Candida antarctica,* z. B. Novozym^{®} 435 von der Fa. Sigma-Aldrich bzw. Merck oder Lipozym^{®}435 von der Fa. Strem Chemicals, Inc.) vorgelegt.

Das Reaktionsgemisch wird unter Rühren bei 70°C und unter Vakuum (< 500 mbar) für 7 h zur Reaktion gebracht. Zur Reaktionskontrolle werden Proben nach jeweils 0,5 h, 1 h, 2 h, 3 h, 4 h, 5 h und 7 h entnommen und mittels GC analysiert. Anschließend wird das Enzym abfiltriert und das Produkt 3-BHB-Oleylester im Vakuum durch mehrfache Destillation erhalten. Der erhaltene Rückstand wird bei Bedarf für 2 bis 4 h im Hochvakuum gedämpft (Dampftemperatur 160 °C). Es wird reiner 3-BHB-Oleylester erhalten.

**Tabelle: Umsatz/Zeit-Verlauf bei der Herstellung von 3-BHB-Oleylester (50 °C, 50 - 60 mbar, 24 h, 1 Gew.-% Enzym)**

| Reaktionszeit [h] | | 0 | 0,5 | 1 | 2 | 3 | 4 | 5 | 7 |
|---|---|---|---|---|---|---|---|---|---|
| | Peak bei [min] | [%] | [%] | [%] | [%] | [%] | [%] | [%] | [%] |
| 3-BHB-Ethyl | 10 | 29,4 | 21,6 | 18,7 | 15,1 | 12,7 | 11,5 | 10,1 | 7,7 |
| Dimere | 20 | 0,0 | 0,4 | 0,5 | 0,6 | 0,6 | 0,6 | 0,7 | 0,8 |
| Oleylalkohol | 27 | 70,6 | 56,8 | 48,9 | 40,5 | 34,2 | 29,7 | 28,5 | 24,8 |
| 3-BHB-Oleyl | 31 | 0,0 | 21,2 | 31,9 | 43,8 | 52,5 | 58,2 | 60,7 | 66,7 |

### Herstellung von weiteren 3-BHB-Fettalkoholestern gemäß Syntheseroute (A)

Die vorstehenden enzymkatalysierten Synthesen werden entsprechend auch mit weiteren Fettalkoholen durchgeführt (nämlich jeweils mit Cetylalkohol, Margarylalkohol, Stearylalkohol, Behenylalkohol, Melissylalkohol, Palmitoleylalkohol und Linoleylalkohol). Es werden die entsprechenden 3-BHB-Fettalkoholester jeweils als Reinstoffe erhalten.

### Weitere von Herstellung von 3-BHB-Fettalkoholestern (nicht erfindungsgemäß)

Die vorangehenden Versuche werden wiederholt, jedoch mit Natriummethylat (NaOMe) als Katalysator (1 Gew.-%) anstelle des Enzyms, 40 Mol-% Überschuss 3-BHB-Ethylester und bei Temperaturen zwischen 100 und 120 °C. Es werden vergleichbare Ergebnisse erhalten. Aufreinigung und Trennung erfolgen in gleicher Weise.

### Herstellung von 3-BHB-Decylester gemäß Syntheseroute (B)

### 1. Stufe: Synthese des acetylierten 3-BHB-Anhydrids

In einem 1.000-ml-Mehrhalskolben mit Dephlegmator (Partialkondensator) und Destillationsbrücke werden 25 g (R)/(S)-3-Hydroxybuttersäure in 95 g Essigsäure vorgelegt. Zu der Reaktionsmischung werden bei 80 °C unter N₂-Atmosphäre 90 g Essigsäureanhydrid innerhalb von einer Stunde zu getropft. Das Reaktionsgemisch wird bei 80 °C für weitere 4 bis 5 Stunden gerührt. Es wird 3-Acetoxybuttersäureanhydrid (= acetyliertes 3-Hydroxybuttersäureanhydrid) gebildet.

### 2. Stufe: Umsetzung des acetylierten 3-BHB-Anhydrids mit Fettalkohol

Zu der Reaktionsmischung werden dann bei 80 °C 30 g 1-Decanol gegeben und für 8 bis 10 Stunden gerührt. Das Reaktionsprodukt der zweiten Stufe ist ein Fettalkoholester der 3-Acetoxybuttersäure (d. h. also mit anderen Worten ein Fettalkoholester der acetylierten 3-Hydroxybuttersäure) und ein Intermediat bzw. Zwischenprodukt für die Herstellung des entsprechenden 3-BHB-Fettalkoholesters, ist aber selbst auch pharmazeutisch für denselben Verwendungszweck einsetzbar bzw. wirksam.

Die gebildeten Nebenprodukte (Essigsäure aus der ersten Stufe und 3-Acetoxybuttersäure aus der zweiten Stufe) werden im Vakuum (< 50 mbar) bei 100 bis 120 °C abdestilliert; man erhält reinen Fettalkoholester der 3-Acetoxybuttersäure. Die Charakterisierung erfolgt mittels GC, GPC und GC-MS.

### 3. Stufe: Enzymkatalysierte Hydrolyse der Acetylgruppe

Ein Teil dieses Zwischenprodukts aus der zweiten Stufe (d. h. Fettalkoholester der 3-Acetoxybuttersäure) wird anschließend einer Hydrolyse der Acetylgruppe unterzogen (partielle bzw. selektive Hydrolyse in Gegenwart von Enzym). Hierzu wird das Reaktionsprodukt aus der zweiten Stufe in wässrigem Milieu in Gegenwart von immobilisiertem Enzym (CALB-Lipase auf Polymerträger, abgeleitet von *Candida antarctica,* erster Ansatz: Novozym^{®}435 von der Fa. Sigma-Aldrich bzw. Merck und zweiter Ansatz: Lipozym^{®}435 von der Fa. Strem Chemicals, Inc.) für 8 Stunden bei 50 °C umgesetzt. Nach Abtrennung des Enzyms und anschließender destillativer Aufreinigung wird als Hydrolyseprodukt der betreffende Fettalkoholester der 3-Hydroxybuttersäure erhalten, d. h. 3-Hydroxybuttersäuredecylester. Die Charakterisierung erfolgt mittels GC, GPC und GC-MS.

Der verbleibende Teil des Zwischenprodukts wird für nachfolgende Wirksamkeitsversuche eingesetzt.

### Weitere Herstellung von Herstellung von 3-BHB-Decylester gemäß Syntheseroute (B)

Der vorangehende Versuch wird wiederholt, jedoch wird nach der Umsetzung von (R)/(S)-3-Hydroxybuttersäureanhydrid zunächst das in der ersten Stufe gebildete Nebenprodukt (Essigsäure) im Vakuum (< 50 mbar) bei 100 bis 120 °C destillativ entfernt und es wird reines 3-Acetoxybuttersäureanhydrid erhalten. Die Charakterisierung erfolgt mittels GC, GPC und GC-MS.

Anschließend wird das reine 3-Acetoxybuttersäureanhydrid mit 1-Decanol umgesetzt, aufgereinigt und analysiert (wie im vorherigen Versuch beschrieben), sodass ein reiner Fettalkoholester der 3-Acetoxybuttersäure erhalten wird.

Das Reaktionsprodukts (d. h. Fettalkoholester der 3-Acetoxybuttersäure) wird anschließend, wie im vorherigen Versuch beschrieben, hydrolysiert, sodass ein Fettalkoholester der 3-Hydroxybuttersäure erhalten wird d. h. 3-Hydroxybuttersäuredecylester. Die Charakterisierung erfolgt mittels GC, GPC und GC-MS.

### Herstellung von 3-BHB-Oleylester gemäß Syntheseroute (B)

### 1. Stufe: Synthese des acetylierten 3-BHB-Anhydrids

In einem 1.000-ml-Mehrhalskolben mit Dephlegmator (Partialkondensator) und Destillationsbrücke werden 25 g (R)/(S)-3-Hydroxybuttersäure in 95 g Essigsäure vorgelegt. Zu der Reaktionsmischung werden bei 80 °C unter N₂-Atmosphäre 90 g Essigsäureanhydrid innerhalb von einer Stunde zu getropft. Das Reaktionsgemisch wird bei 80 °C für weitere 4 bis 5 Stunden gerührt. Es wird 3-Acetoxybuttersäureanhydrid (= acetyliertes 3-Hydroxybuttersäureanhydrid) gebildet.

### 2. Stufe: Umsetzung des acetylierten 3-BHB-Anhydrids mit Fettalkohol

Zu der Reaktionsmischung werden dann bei 80 °C 52 g Oleylalkohol gegeben und für 8 bis 10 Stunden gerührt. Das Reaktionsprodukt der zweiten Stufe ist ein Fettalkoholester der 3-Acetoxybuttersäure (d. h. also mit anderen Worten ein Oleylalkoholester der acetylierten 3-Hydroxybuttersäure) und ein Intermediat bzw. Zwischenprodukt für die Herstellung des entsprechenden 3-BHB-Fettalkoholesters, ist aber selbst auch pharmazeutisch für denselben Verwendungszweck einsetzbar bzw. wirksam.

Die gebildeten Nebenprodukte (Essigsäure aus der ersten Stufe und 3-Acetoxybuttersäure aus der zweiten Stufe) werden im Vakuum (< 50 mbar) bei 100 bis 120 °C abdestilliert; man erhält reinen Fettalkoholester der 3-Acetoxybuttersäure. Die Charakterisierung erfolgt mittels GC, GPC und GC-MS.

### 3. Stufe: Enzymkatalysierte Hydrolyse der Acetylgruppe

Ein Teil dieses Zwischenprodukts aus der zweiten Stufe (d. h. Oleylalkoholester der 3-Acetoxybuttersäure) wird anschließend einer Hydrolyse der Acetylgruppe unterzogen (partielle bzw. selektive Hydrolyse in Gegenwart von Enzym). Hierzu wird das Reaktionsprodukt aus der zweiten Stufe in wässrigem Milieu in Gegenwart von immobilisiertem Enzym (CALB-Lipase auf Polymerträger, abgeleitet von *Candida antarctica,* erster Ansatz: Novozym^{®}435 von der Fa. Sigma-Aldrich bzw. Merck und zweiter Ansatz: Lipozym^{®}435 von der Fa. Strem Chemicals, Inc.) für 8 Stunden bei 50 °C umgesetzt. Nach Abtrennung des Enzyms und anschließender destillativer Aufreinigung wird als Hydrolyseprodukt der betreffende Fettalkoholester der 3-Hydroxybuttersäure erhalten, d. h. 3-Hydroxybuttersäureoleylester. Die Charakterisierung erfolgt mittels GC, GPC und GC-MS.

Der verbleibende Teil des Zwischenprodukts wird für nachfolgende Wirksamkeitsversuche eingesetzt.

### Weitere Herstellung von 3-BHB-Oleylester gemäß Syntheseroute (B)

Der vorangehende Versuch wird wiederholt, jedoch wird nach der Umsetzung von (R)/(S)-3-Hydroxybuttersäureanhydrid zunächst das in der ersten Stufe gebildete Nebenprodukt (Essigsäure) im Vakuum (< 50 mbar) bei 100 bis 120 °C destillativ entfernt und es wird reines 3-Acetoxybuttersäureanhydrid erhalten. Die Charakterisierung erfolgt mittels GC, GPC und GC-MS.

Anschließend wird das reine 3-Acetoxybuttersäureanhydrid mit Oleylalkohol umgesetzt, aufgereinigt und analysiert (wie im vorherigen Versuch beschrieben), sodass ein reiner Fettalkoholester der 3-Acetoxybuttersäure erhalten wird.

Das Reaktionsprodukts (d. h. Oleylalkoholester der 3-Acetoxybuttersäure) wird anschließend, wie im vorherigen Versuch beschrieben, hydrolysiert, sodass ein Oleylalkoholester der 3-Hydroxybuttersäure erhalten wird d. h. 3-Hydroxybuttersäureoleylester. Die Charakterisierung erfolgt mittels GC, GPC und GC-MS.

### Weitere Herstellung von 3-BHB-Fettsäureestern gemäß Syntheseroute (B)

Die vier vorangehenden Versuche werden jeweils wiederholt, jedoch in Anwesenheit eines sauren Katalysators.

In einer ersten Ansatzreihe werden die Umsetzungen von 1-Decanol bzw. Oleylalkohol in Gegenwart von Schwefelsäure (H₂SO₄) als Katalysator und bei Temperaturen zwischen 75 und 110 °C wiederholt. Es werden vergleichbare Ergebnisse erhalten. Aufreinigung, Trennung bzw. Fraktionierung und Hydrolyse erfolgen in gleicher Weise.

In einer zweiten Ansatzreihe werden die Umsetzungen von 1-Decanol bzw. Oleylalkohol in Gegenwart von Salzsäure (HCl) als Katalysator und bei Temperaturen zwischen 75 und 110 °C wiederholt. Es werden vergleichbare Ergebnisse erhalten. Aufreinigung, Trennung bzw. Fraktionierung und Hydrolyse erfolgen in gleicher Weise.

In einer dritten Ansatzreihe werden die Umsetzungen von 1-Decanol bzw. Oleylalkohol in Gegenwart von Phosphorsäure (H₃PO₄) als Katalysator und bei Temperaturen zwischen 75 und 110 °C wiederholt. Es werden vergleichbare Ergebnisse erhalten. Aufreinigung, Trennung bzw. Fraktionierung und Hydrolyse erfolgen in gleicher Weise.

### Herstellung von weiteren 3-BHB-Fettalkoholestern gemäß Syntheseroute (B)

Die vorstehenden Synthesen (sowohl autokatalytisch als auch mineralsäurekatalysiert) werden entsprechend auch für weitere Fettalkohohole durchgeführt (nämlich jeweils für Cetylalkohol, Margarylalkohol, Stearylalkohol, Behenylalkohol, Melissylalkohol, Palmitoleylalkohol und Linoleylalkohol). Es werden die entsprechenden 3-BHB-Fettalkoholester jeweils als Reinstoffe erhalten.

### Physiologische Anwendungsversuche: in-vitro-Verdauversuche Verdauversuche (Spalt- bzw. Spaltungsversuche) von erfindunasaemäßen 3-BHB-Fettalkoholestern (d. h. Fettalkoholestern der 3-Hydroxybuttersäure)

Mittels Spaltungsversuchen wird gezeigt, dass erfindungsgemäß hergestellte 3-BHB-Fettalkoholester, einschließlich der Reaktionsnebenprodukte wie Dimere etc., sowie deren acylierte Derivate im menschlichen gastrointestinalen Trakt gespalten werden können.

Als Testsubstanz werden jeweils einerseits aufgereinigte, nach dem erfindungsgemäßen Verfahren erhaltene 3-BHB-Fettalkoholester (d. h. sowohl erhalten durch das Verfahren gemäß Syntheseroute (A) als auch durch das Verfahren gemäß Syntheseroute (B)) und andererseits aufgereinigte, nach dem erfindungsgemäßen Verfahren gemäß Syntheseroute (B) als Reaktionszwischenprodukte erhaltene Fettalkoholester von acetylierter 3-BHB (3-Acetoxybuttersäure) eingesetzt.

### Getestete Fettalkoholester:

- 3-BHB-Decylester
- Decylester von acetylierter 3-BHB (Decylalkoholester der 3-Acetoxybuttersäure)
- 3-BHB-Oleylester
- Oleylester von acetylierter 3-BHB (Oleylalkoholester der 3-Acetoxybuttersäure)
- 3-BHB-Cetylester
- 3-BHB-Margarylester
- 3-BHB-Stearylester
- 3-BHB-Behenylester
- 3-BHB-Melissylester
- 3-BHB-Palmitoleylester
- 3-BHB-Linoleylester

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

### Weitere Verdauversuche (Spaltungsversuche) von erfindungsgemäßen 3-BHB-Fettalkoholestern (d. h. Fettalkoholestern der 3-Hydroxybuttersäure) Spaltungsversuche mit Pankreatin

Jeweils 2 g der wie zuvor beschrieben hergestellten Fettalkoholester von 3-Hydroxybuttersäure (d. h. sowohl erhalten durch das Verfahren gemäß Syntheseroute (A), als auch durch das Verfahren gemäße Syntheseroute (B)) bzw. deren acetylierte Derivate werden jeweils in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung des 3-BHB-Fettalkoholesters bzw. seines Acylderivats zu der freien Säure). Der Umsatz/ZeitVerlauf der wässrigen Spaltung des erfindungsgemäßen Estergemischs mittels Pankreatin, einschließlich Zunahme der Säurezahl über die Zeit, belegt die gewünschte Zersetzung des Eduktgemischs zu der freien Säure. Dies wird durch entsprechende Analytik bestätigt. Der Versuch belegt, dass die erfindungsgemäßen Fettalkoholester von 3-Hydroxybuttersäure (d. h. sowohl erhalten durch das Verfahren gemäß Syntheseroute (A), als auch durch das Verfahren gemäße Syntheseroute (B)) bzw. deren acetylierte Derivate geeignete physiologische Präkursoren für 3-Hydroxybuttersäure für die entsprechenden Ketokörpertherapien darstellen.

Die zuvor geschilderten Spaltungsversuche belegen, dass die Fettalkoholester der 3-Hydroxybuttersäure bzw. deren Acylderivate effiziente Präkursoren bzw. Metabolite der freien 3-Hydroxybuttersäure bzw. deren Salzen darstellen, insbesondere im Hinblick auf ihre beabsichtigte Wirkung, welche in physiologisch verträglicher bzw. physiologisch kompatibler Form vorliegen.

## Patentansprüche

1. Verfahren zur Herstellung von Fettalkoholestern der 3-Hydroxybuttersäure (beta-Hydroxybuttersäure, BHB bzw. 3-BHB),
(A) wobei gemäß einer (ersten) Syntheseroute (A) mindestens eine Verbindung der allgemeinen Formel (la)
CH₃ - CH(OH) - CH₂ - C(O)OR¹ (la)
wobei in der allgemeinen Formel (la) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit mindestens einem Fettalkohol (II), ausgewählt aus C₁₀-C₃₀-Fettalkoholen, insbesondere C₁₀-C₂₄-Fettalkoholen, umgesetzt wird,
wobei die Umsetzung in Abwesenheit von Lösemitteln und/oder ohne jedwedes Lösemittel durchgeführt wird,
wobei die Umsetzung in Gegenwart eines Enzyms als Katalysator durchgeführt wird, wobei der Katalysators nach Umsetzung rezykliert wird, und
wobei bei der Umsetzung gleichzeitig die Verbindung gemäß der allgemeinen Formel (IVa)
R¹ - OH (IVa)
gebildet wird, wobei in der allgemeinen Formel (IVa) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, wobei die Verbindung gemäß der allgemeinen Formel (IVa) der Umsetzung kontinuierlich entzogen wird;
oder aber
(B) wobei gemäß einer (zweiten, zu (A) alternativen) Syntheseroute (B) mindestens eine Verbindung der allgemeinen Formel (Ib)
CH₃-CH(OR²)-CH₂-C(O)-O-C(O)-CH₂-CH(OR²)-CH₃ (Ib)
wobei in der allgemeinen Formel (Ib) der Rest R² eine Acylgruppe, ausgewählt aus -C(O)-CH₃ (Acetylgruppe) oder -C(O)-C₂H₅ (Propionylgruppe), bevorzugt -C(O)-CH₃ (Acetylgruppe), darstellt,
mit mindestens einem Fettalkohol (II), ausgewählt aus C₁₀-C₃₀-Fettalkoholen, insbesondere C₁₀-C₂₄-Fettalkoholen, umgesetzt wird, gefolgt von einer Hydrolyse der Acylgruppe;
so dass als Reaktionsprodukt (III) jeweils ein C₁₀-C₃₀-Fettalkoholester der 3-Hydroxybuttersäure, insbesondere ein C₁₀-C₂₄-Fettalkoholester der 3-Hydroxybuttersäure, erhalten wird.

2. Verfahren nach Anspruch 1,
wobei gemäß Syntheseroute (A) das Enzym ausgewählt wird aus Synthetasen (Ligasen), Katalasen, Esterasen, Lipasen und deren Kombinationen; und/oder
wobei gemäß Syntheseroute (A) das Enzym sich ableitet von *Candida antarctica, Mucor miehei* (*Rhizomucor miehei*), *Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus;* und/oder
wobei gemäß Syntheseroute (A) das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
wobei gemäß Syntheseroute (A) das Enzym nach der Umsetzung rezykliert wird; und/oder
wobei gemäß Syntheseroute (A) die Umsetzung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
wobei gemäß Syntheseroute (A) das Enzym in Mengen, bezogen auf die Gesamtmenge der Ausgangsverbindungen (la) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
wobei gemäß Syntheseroute (A) die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

3. Verfahren nach Anspruch 1 oder Anspruch 2,
wobei gemäß Syntheseroute (A) die Verbindung der allgemeinen Formel (la), bezogen auf die Hydroxylgruppen des Fettalkohols (II), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei gemäß Syntheseroute (A) die Verbindung der allgemeinen Formel (la) und der Fettalkohol (II) in einem Molverhältnis von Verbindung der allgemeinen Formel (Ia) / Fettalkohol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

4. Verfahren nach Anspruch 1,
wobei gemäß Syntheseroute (B) in der allgemeinen Formel (Ib) der Rest R² eine Gruppe - C(O) - CH₃ (Acetylgruppe) darstellt und/oder
wobei gemäß Syntheseroute (B) als Verbindung der allgemeinen Formel (Ib) die Verbindung der Formel CH₃- CH(OAc) - CH₂ - C(O) - O - C(O) - CH₂ - CH(OAc) - CH₃, wobei der Rest Ac eine Acetylgruppe darstellt, eingesetzt wird; und/oder
wobei gemäß Syntheseroute (B) die Umsetzung in Abwesenheit von Lösemitteln und/oder ohne jedwedes Lösemittel durchgeführt wird; und/oder
wobei gemäß Syntheseroute (B) die Umsetzung autokatalytisch oder in Gegenwart eines Katalysators, insbesondere einer mineralischen Säure, vorzugsweise autokatalytisch, durchgeführt wird; und/oder
wobei gemäß Syntheseroute (B) die Umsetzung in Gegenwart eines Katalysators, insbesondere einer mineralischen Säure, durchgeführt wird; insbesondere wobei gemäß Syntheseroute (B) der Katalysator und/oder die mineralische Säure ausgewählt wird/werden aus Schwefelsäuren, Halogenwasserstoffsäuren, Phosphorsäuren und deren Mischungen; und/oder
wobei gemäß Syntheseroute (B) die Umsetzung bei Temperaturen im Bereich von 20 °C bis 150 °C, insbesondere im Bereich von 50 °C bis 140 °C, vorzugsweise im Bereich von 60 °C bis 130 °C, besonders bevorzugt im Bereich von 70 °C bis 125 °C, ganz besonders bevorzugt im Bereich von 75 °C bis 110 °C, durchgeführt wird; und/oder
wobei gemäß Syntheseroute (B) die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird; und/oder
wobei gemäß Syntheseroute (B) die Verbindung der allgemeinen Formel (Ib), bezogen auf die Hydroxylgruppen des Fettalkohols (II), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei gemäß Syntheseroute (B) die Verbindung der allgemeinen Formel (Ib) und der Fettalkohol (II) in einem Molverhältnis von Verbindung der allgemeinen Formel (Ib) / Fettalkohol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

5. Verfahren nach Anspruch 1 oder Anspruch 4,
wobei gemäß Syntheseroute (B) die Hydrolyse der Acylgruppe, insbesondere der Acetylgruppe, in Gegenwart eines Katalysators, vorzugsweise eines Enzyms, erfolgt;
insbesondere wobei das Enzym ausgewählt wird aus Synthetasen (Ligasen), Katalasen, Esterasen, Lipasen und deren Kombinationen; und/oder
insbesondere wobei das Enzym sich ableitet von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus;* und/oder
insbesondere wobei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
insbesondere wobei das Enzym in Mengen, bezogen auf die Gesamtmenge der zu hydrolysierenden Verbindung, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
insbesondere wobei das Enzym nach der Umsetzung rezykliert wird; und/oder
wobei gemäß Syntheseroute (B) die Hydrolyse der Acylgruppe, insbesondere der Acetylgruppe, bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
wobei gemäß Syntheseroute (B) die Hydrolyse der Acylgruppe, insbesondere der Acetylgruppe, bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird;
wobei gemäß Syntheseroute (B) die Hydrolyse der Acylgruppe, insbesondere der Acetylgruppe, in Gegenwart von Wasser durchgeführt wird.

6. Verfahren nach einem der vorangehenden Ansprüche,
wobei der Fettalkohol (II) der allgemeinen Formel (II')
R³ - OH (II')
entspricht, wobei der Rest R³ einen linearen oder verzweigten, gesättigten oder ein- oder mehrfach ungesättigten aliphatischen C₁₀-C₃₀-Alkylrest, vorzugsweise C₁₀-C₂₄-Alkylrest, darstellt, insbesondere wobei die Hydroxylfunktion (OH-Funktion) primär und/oder endständig ist.

7. Verfahren nach Anspruch 6,
wobei der Rest R³ einen linearen, gesättigten oder ein- oder mehrfach ungesättigten aliphatischen C₁₀-C₂₄-Alkylrest darstellt, insbesondere wobei die Hydroxylfunktion (OH-Funktion) primär und/oder endständig ist; und/oder
wobei der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis-*9-Hexadecen-1-ylrest (Palmitoleylrest), einen *cis*-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octadecadien-1-ylrest (Linoleylrest) oder einen 6,9,12-Octadecatrien-1-ylrest (γ-Linolenylrest), bevorzugt einen cis-9-Octadecen-1-ylrest (Oleylrest), darstellt.

8. Verfahren nach einem der vorangehenden Ansprüche,
wobei der Fettalkohol (II) ausgewählt ist aus linearen oder verzweigten, gesättigten oder ein- oder mehrfach ungesättigten aliphatischen C₁₀-C₃₀-Fettalkoholen, insbesondere C₁₀-C₂₄-Fettalkoholen, bevorzugt mit primärer und/oder endständiger Hydroxylfunktion (OH-Funktion); und/oder
wobei der Fettalkohol (II) ausgewählt ist aus ein linearen, gesättigten oder ein- oder mehrfach ungesättigten, aliphatischen einwertigen und bevorzugt primären C₁₀-C₃₀-Fettalkoholen, insbesondere linearen, gesättigten oder ein- oder mehrfach ungesättigten, aliphatischen einwertigen und bevorzugt primären C₁₀-C₂₄-Fettalkoholen; und/oder
wobei der Fettalkohol (II) ausgewählt ist aus der Gruppe von 1-Decanol, 1-Dodecanol (Laurylalkohol), 1-Tetradecanol (Myristylalkohol), 1-Hexadecanol (Cetylalkohol), 1-Heptadecanol (Margarylalkohol), 1-Octadecanol (Stearylalkohol), 1-Eicosanol (Arachidylalkohol), 1-Docosanol (Behenylalkohol), 1-Tetracosanol (Ligocerylalkohol), 1-Hexacosanol (Cerylalkohol), 1-Octacosanol (Montanylalkohol), 1-Tricontanol (Melissylalkohol), *cis*-9-Hexadecen-1-ol (Palmitoleylalkohol), *cis*-9-Octadecen-1-ol (Oleylalkohol), *trans*-9-Octadecen-1-ol (Elaidylalkohol), *cis*-11-Octadecen-1-ol, *cis,cis*-9,12-Octadecadien-1-ol (Linoleylalkohol), 6,9,12-Octadecatrien-1-ol (γ-Linolenylalkohol), und deren Mischungen, bevorzugt cis-9-Octadecen-1-ol (Oleylalkohol).

9. Fettalkoholester der 3-Hydroxybuttersäure,
wobei der Fettalkoholester der 3-Hydroxybuttersäure der allgemeinen Formel (III')
CH₃- CH(OH) - CH₂ - C(O)OR³ (III')
entspricht,
wobei der Rest R³ einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Tricontanylrest (Melissylrest), einen *cis-*9-Hexadecen-1-ylrest (Palmitoleylrest), einen *cis*-9-Octadecen-1-ylrest (Oleylrest), einen trans-9-Octadecen-1-ylrest (Elaidylrest), einen *cis*-11-Octadecen-1-ylrest, einen *cis,cis-*9,12-Octadecadien-1-ylrest (Linoleylrest) oder einen 6,9,12-Octadecatrien-1-ylrest (γ-Linolenylrest), bevorzugt einen cis-9-Octadecen-1-ylrest (Oleylrest), darstellt.

10. Fettalkoholester der acylierten 3-Hydroxybuttersäure (3-Acyloxybuttersäure),
wobei der Fettalkoholester der acylierten 3-Hydroxybuttersäure (3-Acyloxybuttersäure) ein Fettalkoholester der acylierten 3-Hydroxybuttersäure (3-Acyloxybuttersäure) der allgemeinen Formel (IIIa)
CH₃- CH(OR²) - CH₂ - C(O)OR³ (IIIa)
ist,
wobei in der allgemeinen Formel (IIIa)
• R² eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
• der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis-*9-Hexadecen-1-ylrest (Palmitoleylrest), einen *cis*-9-Octadecen-1-ylrest (Oleylrest), einen *trans*-9-Octa-decen-1-ylrest (Elaidylrest), einen *cis*-11-Octadecen-1-ylrest, einen *cis,cis*-9,12-Octa-decadien-1-ylrest (Linoleylrest) oder einen 6,9,12-Octadecatrien-1-ylrest (γ-Linolenylrest), bevorzugt einen *cis*-9-Octadecen-1-ylrest (Oleylrest), darstellt.

11. Pharmazeutische Zusammensetzung, insbesondere Arzneimittel oder Medikament, umfassend einen Fettalkoholester der 3-Hydroxybuttersäure gemäß Anspruch 9 oder einen Fettalkoholester der acylierten 3-Hydroxybuttersäure (3-Acyloxybuttersäure) gemäß Anspruch 10.

12. Pharmazeutische Zusammensetzung nach Anspruch 11 zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

13. Fettalkoholester der 3-Hydroxybuttersäure Anspruch 9 oder Fettalkoholester der acylierten 3-Hydroxybuttersäure (3-Acyloxybuttersäure) gemäß Anspruch 10 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

14. Verwendung eines Fettalkoholesters der 3-Hydroxybuttersäure gemäß Anspruch 9 oder eines Fettalkoholesters der acylierten 3-Hydroxybuttersäure (3-Acyloxybuttersäure) gemäß Anspruch 10 zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

15. Nahrungsmittel- und/oder Lebensmittelerzeugnis, umfassend einen Fettalkoholester der 3-Hydroxybuttersäure gemäß Anspruch 9 oder einen Fettalkoholester der acylierten 3-Hydroxybuttersäure (3-Acyloxybuttersäure) gemäß Anspruch 10.

16. Fettalkoholester der 3-Hydroxybuttersäure zur Verwendung als Arzneimittel oder Medikament,
wobei der Fettalkoholester der 3-Hydroxybuttersäure der allgemeinen Formel (III')
CH₃- CH(OH) - CH₂ - C(O)OR³ (III')
entspricht,
wobei der Rest R³ einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis-*9-Hexadecen-1-ylrest (Palmitoleylrest), einen *cis*-9-Octadecen-1-ylrest (Oleylrest), einen *trans*-9-Octadecen-1-ylrest (Elaidylrest), einen *cis*-11-Octadecen-1-ylrest, einen *cis,cis*-9,12-Octadecadien-1-ylrest (Linoleylrest) oder einen 6,9,12-Octadecatrien-1-ylrest (γ-Linolenylrest), bevorzugt einen *cis*-9-Octadecen-1-ylrest (Oleylrest), darstellt.

17. Fettalkoholester der acylierten 3-Hydroxybuttersäure (3-Acyloxybuttersäure) zur Verwendung als Arzneimittel oder Medikament,
wobei der Fettalkoholester der acylierten 3-Hydroxybuttersäure (3-Acyloxybuttersäure) ein Fettalkoholester der acylierten 3-Hydroxybuttersäure (3-Acyloxybuttersäure) der allgemeinen Formel (IIIa)
CH₃- CH(OR²) - CH₂ - C(O)OR³ (IIIa)
ist,
wobei in der allgemeinen Formel (IIIa)
• R² eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
• der Rest R³ einen 1-Decanylrest, einen 1-Dodecanylrest (Laurylrest), einen 1-Tetradecanylrest (Myristylrest), einen 1-Hexadecanylrest (Cetylrest), einen 1-Heptadecanylrest (Margarylrest), einen 1-Octadecanylrest (Stearylrest), einen 1-Eicosanylrest (Arachidylrest), einen 1-Docosanylrest (Behenylrest), einen 1-Tetracosanylrest (Ligocerylrest), einen 1-Hexacosanylrest (Cerylrest), einen 1-Octacosanylrest (Montanylrest), einen 1-Tricontanylrest (Melissylrest), einen *cis-*9-Hexadecen-1-ylrest (Palmitoleylrest), einen *cis*-9-Octadecen-1-ylrest (Oleylrest), einen *trans*-9-Octa-decen-1-ylrest (Elaidylrest), einen *cis*-11-Octadecen-1-ylrest, einen *cis,cis-*9,12-Octa-decadien-1-ylrest (Linoleylrest) oder einen 6,9,12-Octadecatrien-1-ylrest (γ-Linolenylrest), bevorzugt einen *cis-*9-Octadecen-1-ylrest (Oleylrest), darstellt.

## Claims

1. Process for the preparation of fatty alcohol esters of 3-hydroxybutyric acid (beta-hydroxybutyric acid, BHB and/or 3-BHB),
(A) wherein, according to a (first) synthesis route (A), at least one compound of the general formula (Ia)
CH₃ - CH(OH) - CH₂ - C(O)OR¹ (Ia)
wherein, in the general formula (Ia), the residue R¹ is hydrogen or a C₁-C₄-alkyl, in particular a C₁-C₄-alkyl, preferably methyl or ethyl, particularly preferably ethyl,
is reacted with at least one fatty alcohol (II) selected from C₁₀-C₃₀-fatty alcohols, in particular C₁₀-C₂₄-fatty alcohols,
wherein the reaction is carried out in the absence of solvents and/or without any solvent,
wherein the reaction is carried out in the presence of an enzyme as catalyst, wherein the catalyst is recycled after the reaction, and
wherein, during the reaction, the compound according to the general formula (IVa)
R¹ - OH (IVa)
is formed, wherein, in the general formula (IVa), the residue R¹ is hydrogen or a C₁-C₄-alkyl, in particular a C₁-C₄-alkyl, preferably methyl or ethyl, particularly preferably ethyl, wherein the compound according to the general formula (IVa) is continuously withdrawn from the reaction;
or alternatively
(B) wherein, according to a (second, alternative to (A)) synthesis route (B), at least one compound of the general formula (Ib)
CH₃-CH(OR²)-CH₂ -C(O)- O -C(O)-CH₂ -CH(OR²)-CH₃ (Ib)
wherein, in the general formula (Ib), the residue R² is an acyl group selected from - C(O) - CH₃ (acetyl group) or - C(O) - C₂H₅ (propionyl group), preferably - C(O) - CH₃ (acetyl group),
is reacted with at least one fatty alcohol (II) selected from C₁₀-C₃₀-fatty alcohols, in particular C₁₀-C₂₄-fatty alcohols, followed by hydrolysis of the acyl group;
so that a C₁₀-C₃₀-fatty alcohol ester of 3-hydroxybutyric acid, in particular a C₁₀-C₂₄-fatty alcohol ester of 3-hydroxybutyric acid, is obtained in each case as reaction product (III).

2. Process according to claim 1,
wherein, according to synthesis route (A), the enzyme is selected from synthetases (ligases), catalases, esterases, lipases and combinations thereof; and/or
wherein, according to synthesis route (A), the enzyme is derived from *Candida antarctica, Mucor miehei* (*Rhizomucor miehei*)*, Thermomyces lanuginosus, Candida rugosa, Aspergillus oryzae, Pseudomonas cepacia, Pseudomonas fluorescens, Rhizopus delemar* and *Pseudomonas* sp. and combinations thereof, preferably from *Candida antarctica, Mucor miehei* (*Rhizomucor miehei*) and *Thermomyces lanuginosus;* and/or
wherein, according to synthesis route (A), the enzyme is used in immobilized form, in particular immobilized on a support, preferably on a polymeric support, preferably on a polymeric organic support, particularly preferably with hydrophobic properties, very particularly preferably on a poly(meth)acrylic resin-based support; and/or
wherein, according to synthesis route (A), the enzyme is recycled after the reaction; and/or
wherein, according to synthesis route (A), the reaction is carried out in the presence of an enzyme as catalyst at temperatures in the range from 10 °C to 80 °C, in particular in the range from 20 °C to 80 °C, preferably in the range from 25 °C to 75 °C, particularly preferably in the range from 45 °C to 75 °C, very particularly preferably in the range from 50 °C to 70 °C; and/or
wherein, according to synthesis route (A), the enzyme is used in amounts, based on the total amount of the starting compounds (Ia) and (II), in the range from 0.001 wt.-% to 20 wt.-%, in particular in the range from 0.01 wt.-% to 15 wt.-%, preferably in the range from 0.1 wt.-% to 15 wt.-%, preferably in the range from 0.5 wt.-% to 10 wt.-%; and/or
wherein, according to synthesis route (A), the reaction is carried out in the presence of an enzyme as catalyst at a pressure in the range from 0.0001 bar to 10 bar, in particular in the range from 0.001 bar to 5 bar, preferably in the range from 0.01 bar to 2 bar, particularly preferably in the range from 0.05 bar to 1 bar, very particularly at about 1 bar.

3. Process according to claim 1 or claim 2,
wherein, according to synthesis route (A), the compound of the general formula (Ia), based on the hydroxyl groups of the fatty alcohol (II), is used in molar amounts in a range from equimolar amount up to a molar excess of 200 mol-%, in particular in a range from equimolar amount up to a molar excess of 150 mol-%, preferably in a range from equimolar amount up to a molar excess of 100 mol-%; and/or
wherein, according to synthesis route (A), the compound of the general formula (la) and the fatty alcohol (II) are used in a molar ratio of compound of the general formula (Ia) / fatty alcohol (II) in a range from 1 : 1 to 10 : 1, in particular in a range from 2 : 1 to 8 : 1, preferably in a range from 3 : 1 to 6 : 1.

4. Process according to claim 1,
wherein, according to synthesis route (B), in the general formula (Ib), the residue R² is a group - C(O) - CH₃ (acetyl group) and/or
wherein, according to synthesis route (B), the compound of the general formula (Ib) used is the compound of the formula CH₃ - CH(OAc) - CH₂ - C(O) - O - C(O) - CH₂ -CH(OAc) - CH₃, wherein the residue Ac is an acetyl group; and/or
wherein, according to synthesis route (B), the reaction is carried out in the absence of solvents and/or without any solvent; and/or
wherein, according to synthesis route (B), the reaction is carried out autocatalytically or in the presence of a catalyst, in particular a mineral acid, preferably autocatalytically; and/or
wherein, according to synthesis route (B), the reaction is carried out in the presence of a catalyst, in particular a mineral acid; wherein, in particular, according to synthesis route (B), the catalyst and/or the mineral acid is/are selected from sulfuric acids, hydrohalic acids, phosphoric acids, and mixtures thereof; and/or
wherein, according to synthesis route (B), the reaction is carried out at temperatures in the range from 20 °C to 150 °C, in particular in the range from 50 °C to 140 °C, preferably in the range from 60 °C to 130 °C, particularly preferably in the range from 70 °C to 125 °C, very particularly preferably in the range from 75 °C to 110 °C; and/or
wherein, according to synthesis route (B), the reaction is carried out at a pressure in the range from 0.0001 bar to 10 bar, in particular in the range from 0.001 bar to 5 bar, preferably in the range from 0.01 bar to 2 bar, particularly preferably in the range from 0.05 bar to 1 bar, very particularly at about 1 bar; and/or
wherein, according to synthesis route (B), the compound of the general formula (Ib), based on the hydroxyl groups of the fatty alcohol (II), is used in molar amounts in a range from equimolar amount up to a molar excess of 200 mol-%, in particular in a range from equimolar amount up to a molar excess of 150 mol-%, preferably in a range from equimolar amount up to a molar excess of 100 mol-%; and/or
wherein, according to synthesis route (B), the compound of the general formula (Ib) and the fatty alcohol (II) are used in a molar ratio of compound of the general formula (Ib) / fatty alcohol (II) in a range from 1 : 1 to 10 : 1, in particular in a range from 2 : 1 to 8 : 1, preferably in a range from 3 : 1 to 6 : 1.

5. Process according to claim 1 or claim 4,
wherein, according to synthesis route (B), the hydrolysis of the acyl group, in particular the acetyl group, is carried out in the presence of a catalyst, preferably an enzyme;
in particular wherein the enzyme is selected from synthetases (ligases), catalases, esterases, lipases and combinations thereof; and/or
in particular wherein the enzyme is derived from *Candida antarctica, Mucor miehei* (Rhizomucor *miehei*)*, Thermomyces lanuginosus, Candida rugosa, Aspergillus oryzae, Pseudomonas* cepacia, *Pseudomonas fluorescens, Rhizopus delemar* and *Pseudomonas* sp. and combinations thereof, preferably from *Candida antarctica, Mucor miehei* (*Rhizomucor miehei*) and *Thermomyces lanuginosus;* and/or
in particular wherein the enzyme is used in immobilized form, in particular immobilized on a support, preferably on a polymeric support, preferably on a polymeric organic support, particularly preferably with hydrophobic properties, very particularly preferably on a poly(meth)acrylic resin-based support; and/or
in particular wherein the enzyme is used in amounts, based on the total amount of the compound to be hydrolyzed, in the range from 0.001 wt.-% to 20 wt.-%, in particular in the range from 0.01 wt.-% to 15 wt.-%, preferably in the range from 0.1 wt.-% to 15 wt.-%, preferably in the range from 0.5 wt.-% to 10 wt.-%; and/or
in particular wherein the enzyme is recycled after the reaction; and/or
wherein, according to synthesis route (B), the hydrolysis of the acyl group, in particular the acetyl group, is carried out at temperatures in the range from 10 °C to 80 °C, in particular in the range from 20 °C to 80 °C, preferably in the range from 25 °C to 75 °C, particularly preferably in the range from 45 °C to 75 °C, very particularly preferably in the range from 50 °C to 70 °C; and/or
wherein, according to synthesis route (B), the hydrolysis of the acyl group, in particular the acetyl group, is carried out at a pressure in the range from 0.0001 bar to 10 bar, in particular in the range from 0.001 bar to 5 bar, preferably in the range from 0.01 bar to 2 bar, particularly preferably in the range from 0.05 bar to 1 bar, very particularly at about 1 bar;
wherein, according to synthesis route (B), the hydrolysis of the acyl group, in particular the acetyl group, is carried out in the presence of water.

6. Process according to any of the preceding claims,
wherein the fatty alcohol (II) corresponds to the general formula (II')
R³ - OH (II')
wherein the residue R³ is a linear or branched, saturated or mono- or polyunsaturated aliphatic C₁₀-C₃₀-alkyl residue, preferably C₁₀-C₂₄-alkyl residue, in particular wherein the hydroxyl function (OH function) is primary and/or terminal.

7. Process according to claim 6,
wherein the residue R³ is a linear, saturated or mono- or polyunsaturated aliphatic C₁₀-C₂₄-alkyl residue, in particular wherein the hydroxyl function (OH function) is primary and/or terminal; and/or
wherein the residue R³ is a 1-decanyl residue, a 1-dodecanyl residue (lauryl residue), a 1-tetradecanyl residue (myristyl residue), a 1-hexadecanyl residue (cetyl residue), a 1-heptadecanyl residue (margaryl residue), a 1-octadecanyl residue (stearyl residue), a 1-eicosanyl residue (arachidyl residue), a 1-docosanyl residue (behenyl residue), a 1-tetracosanyl residue (ligoceryl residue), a 1-hexacosanyl residue (ceryl residue), a 1-octacosanyl residue (montanyl residue), a 1-tricontanyl residue (melissyl residue), a *cis*-9-hexadecen-1-yl residue (palmitoleyl residue), a *cis*-9-octadecen-1-yl residue (oleyl residue), a *trans*-9-octadecen-1-yl residue (elaidyl residue), a *cis*-11-octadecen-1-yl residue, a *cis,cis*-9,12-octadecadien-1-yl residue (linoleyl residue) or a 6,9,12-octadecatrien-1-yl residue (γ-linolenyl residue), preferably a *cis*-9-octadecen-1-yl residue (oleyl residue).

8. Process according to any of the preceding claims,
wherein the fatty alcohol (II) is selected from linear or branched, saturated or mono- or polyunsaturated aliphatic C₁₀-C₃₀-fatty alcohols, in particular C₁₀-C₂₄-fatty alcohols, preferably with primary and/or terminal hydroxyl function (OH function); and/or
wherein the fatty alcohol (II) is selected from a linear, saturated or mono- or polyunsaturated, aliphatic monovalent and preferably primary C₁₀-C₃₀-fatty alcohols, in particular linear, saturated or mono- or polyunsaturated, aliphatic monovalent and preferably primary C₁₀-C₂₄-fatty alcohols; and/or
wherein the fatty alcohol (II) is selected from the group of 1-decanol, 1-dodecanol (lauryl alcohol), 1-tetradecanol (myristyl alcohol), 1-hexadecanol (cetyl alcohol), 1-heptadecanol (margaryl alcohol), 1-octadecanol (stearyl alcohol), 1-eicosanol (arachidyl alcohol), 1-docosanol (behenyl alcohol), 1-tetracosanol (ligoceryl alcohol), 1-hexacosanol (ceryl alcohol), 1-octacosanol (montanyl alcohol), 1-tricontanol (melissyl alcohol), *cis*-9-hexadecen-1-ol (palmitoleyl alcohol), *cis*-9-octadecen-1-ol (oleyl alcohol), *trans*-9-octadecen-1-ol (elaidyl alcohol), *cis*-11-octadecen-1-ol, *cis,cis*-9,12-octadecadien-1-ol (linoleyl alcohol), 6,9,12-octadecatrien-1-ol (γ-linolenyl alcohol), and mixtures thereof, preferably *cis*-9-octadecen-1-ol (oleyl alcohol).

9. Fatty alcohol ester of 3-hydroxybutyric acid,
wherein the fatty alcohol ester of 3-hydroxybutyric acid corresponds to the general formula (III')
CH₃ - CH(OH) - CH₂ - C(O)OR³ (III')
wherein the residue R³ is a 1-tetradecanyl residue (myristyl residue), a 1-hexadecanyl residue (cetyl residue), a 1-heptadecanyl residue (margaryl residue), a 1-octadecanyl residue (stearyl residue), a 1-eicosanyl residue (arachidyl residue), a 1-docosanyl residue (behenyl residue), a 1-tetracosanyl residue (ligoceryl residue), a 1-tricontanyl residue (melissyl residue), a *cis*-9-hexadecen-1-yl residue (palmitoleyl residue), a *cis*-9-octadecen-1-yl residue (oleyl residue), a *trans*-9-octadecen-1-yl residue (elaidyl residue), a *cis*-11-octadecen-1-yl residue, a *cis,cis*-9,12-octadecadien-1-yl residue (linoleyl residue) or a 6,9,12-octadecatrien-1-yl residue (γ-linolenyl residue), preferably a cis-9-octadecen-1-yl residue (oleyl residue).

10. Fatty alcohol ester of acylated 3-hydroxybutyric acid (3-acyloxybutyric acid),
wherein the fatty alcohol ester of acylated 3-hydroxybutyric acid (3-acyloxybutyric acid) is a fatty alcohol ester of acylated 3-hydroxybutyric acid (3-acyloxybutyric acid) corresponding to the general formula (IIIa)
CH₃ - CH(OR²) - CH₂ - C(O)OR³ (Illa)
wherein in the general formula (IIIa)
• R² is an acyl group selected from - C(O) - CH₃ (acetyl group) or - C(O) - C₂H₅ (propionyl group), preferably - C(O) - CH₃ (acetyl group),
• the residue R³ is a 1-decanyl residue, a 1-dodecanyl residue (lauryl residue), a 1-tetradecanyl residue (myristyl residue), a 1-hexadecanyl residue (cetyl residue), a 1-heptadecanyl residue (margaryl residue), a 1-octadecanyl residue (stearyl residue), a 1-eicosanyl residue (arachidyl residue), a 1-docosanyl residue (behenyl residue), a 1-tetracosanyl residue (ligoceryl residue), a 1-hexacosanyl residue (ceryl residue), a 1-octacosanyl residue (montanyl residue), a 1-tricontanyl residue (melissyl residue), a *cis*-9-hexadecen-1-yl residue (palmitoleyl residue), a *cis*-9-octadecen-1-yl residue (oleyl residue), a *trans*-9-octadecen-1-yl residue (elaidyl residue), a *cis*-11-octadecen-1-yl residue, a *cis,cis*-9,12-octadecadien-1-yl residue (linoleyl residue) or a 6,9,12-octadecatrien-1-yl residue (γ-linolenyl residue), preferably a *cis*-9-octadecen-1-yl residue (oleyl residue).

11. Pharmaceutical composition, in particular drug or medication, comprising a fatty alcohol ester of 3-hydroxybutyric acid according to claim 9 or a fatty alcohol ester of acylated 3-hydroxybutyric acid (3-acyloxybutyric acid) according to claim 10.

12. Pharmaceutical composition according to claim 11 for the prophylactic and/or therapeutic treatment and/or for use in the prophylactic and/or therapeutic treatment of diseases of the human or animal body, in particular diseases related to a disturbance of the energy metabolism, in particular ketone body metabolism, such as in particular cranial-brain trauma, stroke, hypoxias, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipid metabolism diseases such as glucose transporter defect (GLUT1 defect), VL-FAOD and mitochondriopathies such as mitochondrial thiolase defect, Huntington's disease, cancer diseases such as T-cell lymphomas, astrocytomas and glioblastomas, HIV, rheumatic diseases such as rheumatoid arthritis and arthritis urica, diseases of the gastrointestinal tract such as chronic inflammatory bowel diseases, in particular ulcerative colitis and Crohn's disease, lyosomal storage diseases such as sphingolipidoses, in particular Niemann-Pick disease, diabetes mellitus and effects or side effects of chemotherapies.

13. Fatty alcohol ester of 3-hydroxybutyric acid according to claim 9 or fatty alcohol ester of acylated 3-hydroxybutyric acid (3-acyloxybutyric acid) according to claim 10 for use in the prophylactic and/or therapeutic treatment of diseases of the human or animal body, in particular diseases related to a disturbance of the energy metabolism, in particular ketone body metabolism, such as in particular cranial-brain trauma, stroke, hypoxias, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipid metabolism diseases such as glucose transporter defect (GLUT1 defect), VL-FAOD and mitochondriopathies such as mitochondrial thiolase defect, Huntington's disease, cancer diseases such as T-cell lymphomas, astrocytomas and glioblastomas, HIV, rheumatic diseases such as rheumatoid arthritis and arthritis urica, diseases of the gastrointestinal tract such as chronic inflammatory bowel diseases, in particular ulcerative colitis and Crohn's disease, lyosomal storage diseases such as sphingolipidoses, in particular Niemann-Pick disease, diabetes mellitus and effects or side effects of chemotherapies.

14. Use of a fatty alcohol ester of 3-hydroxybutyric acid according to claim 9 or a fatty alcohol ester of acylated 3-hydroxybutyric acid (3-acyloxybutyric acid) according to claim 10 for the preparation of a drug for the prophylactic and/or therapeutic treatment of diseases of the human or animal body, in particular diseases related to a disturbance of the energy metabolism, in particular ketone body metabolism, such as in particular cranial-brain trauma, stroke, hypoxias, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipid metabolism diseases such as glucose transporter defect (GLUT1 defect), VL-FAOD and mitochondriopathies such as mitochondrial thiolase defect, Huntington's disease, cancer diseases such as T-cell lymphomas, astrocytomas and glioblastomas, HIV, rheumatic diseases such as rheumatoid arthritis and arthritis urica, diseases of the gastrointestinal tract such as chronic inflammatory bowel diseases, in particular ulcerative colitis and Crohn's disease, lyosomal storage diseases such as sphingolipidoses, in particular Niemann-Pick disease, diabetes mellitus and effects or side effects of chemotherapies.

15. Food and/or food product, comprising a fatty alcohol ester of 3-hydroxybutyric acid according to claim 9 or a fatty alcohol ester of acylated 3-hydroxybutyric acid (3-acyloxybutyric acid) according to claim 10.

16. Fatty alcohol ester of 3-hydroxybutyric acid for use as drug or medication,
wherein the fatty alcohol ester of 3-hydroxybutyric acid corresponds to the general formula (III')
CH₃ - CH(OH) - CH₂ - C(O)OR³ (III')
wherein the residue R³ is a 1-tetradecanyl residue (myristyl residue), a 1-hexadecanyl residue (cetyl residue), a 1-heptadecanyl residue (margaryl residue), a 1-octadecanyl residue (stearyl residue), a 1-eicosanyl residue (arachidyl residue), a 1-docosanyl residue (behenyl residue), a 1-tetracosanyl residue (ligoceryl residue), a 1-hexacosanyl residue (ceryl residue), a 1-octacosanyl residue (montanyl residue), a 1-tricontanyl residue (melissyl residue), a cis-9-hexadecen-1-yl residue (palmitoleyl residue), a cis-9-octadecen-1-yl residue (oleyl residue), a *trans*-9-octadecen-1-yl residue (elaidyl residue), a *cis*-11-octadecen-1-yl residue, a *cis,cis*-9,12-octadecadien-1-yl residue (linoleyl residue) or a 6,9,12-octadecatrien-1-yl residue (γ-linolenyl residue), preferably a cis-9-octadecen-1-yl residue (oleyl residue).

17. Fatty alcohol ester of acylated 3-hydroxybutyric acid (3-acyloxybutyric acid) for use as drug or medication,
wherein the fatty alcohol ester of acylated 3-hydroxybutyric acid (3-acyloxybutyric acid) is a fatty alcohol ester of acylated 3-hydroxybutyric acid (3-acyloxybutyric acid) corresponding to the general formula (IIIa)
CH₃ - CH(OR²) - CH₂ - C(O)OR³ (Illa)
wherein in the general formula (IIIa)
• R² is an acyl group selected from - C(O) - CH₃ (acetyl group) or - C(O) - C₂H₅ (propionyl group), preferably - C(O) - CH₃ (acetyl group),
• the residue R³ is a 1-decanyl residue, a 1-dodecanyl residue (lauryl residue), a 1-tetradecanyl residue (myristyl residue), a 1-hexadecanyl residue (cetyl residue), a 1-heptadecanyl residue (margaryl residue), a 1-octadecanyl residue (stearyl residue), a 1-eicosanyl residue (arachidyl residue), a 1-docosanyl residue (behenyl residue), a 1-tetracosanyl residue (ligoceryl residue), a 1-hexacosanyl residue (ceryl residue), a 1-octacosanyl residue (montanyl residue), a 1-tricontanyl residue (melissyl residue), a cis-9-hexadecen-1-yl residue (palmitoleyl residue), a cis-9-octadecen-1-yl residue (oleyl residue), a *trans*-9-octadecen-1-yl residue (elaidyl residue), a *cis*-11-octadecen-1-yl residue, a *cis,cis*-9,12-octadecadien-1-yl residue (linoleyl residue) or a 6,9,12-octadecatrien-1-yl residue (γ-linolenyl residue), preferably a *cis*-9-octadecen-1-yl residue (oleyl residue).

## Revendications

1. Procédé pour la préparation d'esters d'alcools gras de l'acide 3-hydroxybutyrique (acide bêta-hydroxybutyrique, BHB et/ou 3-BHB),
(A) où, selon une (première) voie de synthèse (A), au moins un composé de formule générale (la)
CH₃ - CH(OH) - CH₂ - C(O)OR¹ (la)
où, dans la formule générale (la), le reste R¹ représente un atome d'hydrogène ou un groupe alkyle en C₁-C₄, en particulier un groupe alkyle en C₁-C₄, de préférence un groupe méthyle ou éthyle, de manière particulièrement préférée un groupe éthyle,
est mis en réaction avec au moins un alcool gras (II), choisi parmi les alcools gras en C₁₀-C₃₀, en particulier les alcools gras en C₁₀-C₂₄,
où la réaction est réalisée en l'absence de solvants et/ou sans aucun solvant,
où la réaction est réalisée en présence d'une enzyme en tant que catalyseur, où le catalyseur est recyclé après la réaction, et
où, lors de la réaction, le composé selon la formule générale (IVa)
R¹ - OH (IVa)
est formé, où, dans la formule générale (IVa), le reste R¹ représente un atome d'hydrogène ou un groupe alkyle en C₁-C₄, en particulier un groupe alkyle en C₁-C₄, de préférence un groupe méthyle ou éthyle, de manière particulièrement préférée un groupe éthyle, où le composé selon la formule générale (IVa) est retiré de la réaction en continu;
ou bien
(B) où, selon une (deuxième voie de synthèse (B) alternative à (A)), au moins un composé de formule générale (Ib)
CH₃-CH(OR²)-CH₂ -C(O)- O -C(O)-CH₂ -CH(OR²)-CH₃ (Ib)
où, dans la formule générale (Ib), le reste R² représente un groupe acyle choisi parmi - C(O) - CH₃ (groupe acétyle) ou - C(O) - C₂H₅ (groupe propionyle), de préférence - C(O) - CH₃ (groupe acétyle),
est mis en réaction avec au moins un alcool gras (II), choisi parmi les alcools gras en C₁₀-C₃₀, en particulier les alcools gras en C₁₀-C₂₄, suivie d'une hydrolyse du groupe acyle;
de sorte que l'on obtient comme produit de réaction (III), à chaque fois, un ester d'alcool gras en C₁₀-C₃₀ de l'acide 3-hydroxybutyrique, en particulier un ester d'alcool gras en C₁₀-C₂₄ de l'acide 3-hydroxybutyrique.

2. Procédé selon la revendication 1,
où, selon la voie de synthèse (A), l'enzyme est choisie parmi les synthétases (ligases), les catalases, les estérases, les lipases et leurs combinaisons; et/ou
où, selon la voie de synthèse (A), l'enzyme dérive de *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* et *Pseudomonas* sp. ainsi que leurs combinaisons, de préférence de *Candida antarctica, Mucor miehei* (*Rhizomucor miehei*) et *Thermomyces lanuginosus;* et/ou
où, selon la voie de synthèse (A), l'enzyme est utilisée sous forme immobilisée, en particulier immobilisée sur un support, de préférence sur un support polymère, de préférence sur un support organique polymère, de manière particulièrement préférée avec des propriétés hydrophobes, de manière tout particulièrement préférée sur un support à base de résine poly(méth)acrylique; et/ou
où, selon la voie de synthèse (A), l'enzyme est recyclée après la réaction; et/ou
où, selon la voie de synthèse (A), la réaction est réalisée en présence d'une enzyme en tant que catalyseur à des températures dans la plage de 10 °C à 80 °C, en particulier dans la plage de 20 °C à 80 °C, de préférence dans la plage de 25 °C à 75 °C, de manière particulièrement préférée dans la plage de 45 °C à 75 °C, de manière tout particulièrement préférée dans la plage de 50 °C à 70 °C; et/ou
où, selon la voie de synthèse (A), l'enzyme est utilisée en quantités, par rapport à la quantité totale des composés de départ (la) et (II), dans la plage de 0,001 % en poids à 20 % en poids, en particulier dans la plage de 0,01 % en poids à 15 % en poids, de préférence dans la plage de 0,1 % en poids à 15 % en poids, de préférence dans la plage de 0,5 % en poids à 10 % en poids; et/ou
où, selon la voie de synthèse (A), la réaction est réalisée en présence d'une enzyme en tant que catalyseur à une pression dans la plage de 0,0001 bar à 10 bar, en particulier dans la plage de 0,001 bar à 5 bar, de préférence dans la plage de 0,01 bar à 2 bar, de manière particulièrement préférée dans la plage de 0,05 bar à 1 bar, de manière tout particulièrement préférée à environ 1 bar.

3. Procédé selon la revendication 1 ou la revendication 2,
où, selon la voie de synthèse (A), le composé de formule générale (la), par rapport aux groupes hydroxyle de l'alcool gras (II), est utilisé en quantités molaires dans une plage de quantité équimolaire jusqu'à un excès molaire de 200 % en moles, en particulier dans une plage de quantité équimolaire jusqu'à un excès molaire de 150 % en moles, de préférence dans une plage de quantité équimolaire jusqu'à un excès molaire de 100 % en moles; et/ou
où, selon la voie de synthèse (A), le composé de formule générale (la) et l'alcool gras (II) sont utilisés en un rapport molaire composé de formule générale (Ia)/alcool gras (II) dans une plage de 1 : 1 à 10 : 1, en particulier dans une plage de 2 : 1 à 8 : 1, de préférence dans une plage de 3 : 1 à 6 : 1.

4. Procédé selon la revendication 1,
où, selon la voie de synthèse (B), dans la formule générale (Ib), le reste R² représente un groupe - C(O) - CH₃ (groupe acétyle) et/ou
où, selon la voie de synthèse (B), le composé de formule générale (Ib) utilisé est le composé de formule CH₃ - CH(OAc) - CH₂ - C(O) - O - C(O) - CH₂ - CH(OAc) - CH₃, le reste Ac représentant un groupe acétyle; et/ou
où, selon la voie de synthèse (B), la réaction est réalisée en l'absence de solvants et/ou sans aucun solvant; et/ou
où, selon la voie de synthèse (B), la réaction est réalisée de manière autocatalytique ou en présence d'un catalyseur, en particulier d'un acide minéral, de préférence de manière autocatalytique; et/ou
où, la réaction selon la voie de synthèse (B), est réalisée en présence d'un catalyseur, en particulier d'un acide minéral; le catalyseur et/ou l'acide minéral selon la voie de synthèse (B) étant notamment choisis parmi les acides sulfuriques, les acides halogénhydriques, les acides phosphoriques et leurs mélanges;et/ou
où, selon la voie de synthèse (B), la réaction est réalisée à des températures dans la plage de 20 °C à 150 °C, en particulier dans la plage de 50 °C à 140 °C, de préférence dans la plage de 60 °C à 130 °C, de manière particulièrement préférée dans la plage de 70 °C à 125 °C, de manière tout particulièrement préférée dans la plage de 75 °C à 110 °C; et/ou
où, selon la voie de synthèse (B), la réaction est réalisée à une pression dans la plage de 0,0001 bar à 10 bar, en particulier dans la plage de 0,001 bar à 5 bar, de préférence dans la plage de 0,01 bar à 2 bar, de manière particulièrement préférée dans la plage de 0,05 bar à 1 bar, de manière tout particulièrement préférée à environ 1 bar; et/ou
où, selon la voie de synthèse (B), le composé de formule générale (Ib), par rapport aux groupes hydroxyle de l'alcool gras (II), est utilisé en quantités molaires dans une plage de quantité équimolaire jusqu'à un excès molaire de 200 % en moles, en particulier dans une plage de quantité équimolaire jusqu'à un excès molaire de 150 % en moles, de préférence dans une plage de quantité équimolaire jusqu'à un excès molaire de 100 % en moles; et/ou
où, selon la voie de synthèse (B), le composé de formule générale (Ib) et l'alcool gras (II) sont utilisés en un rapport molaire composé de formule générale (Ib)/alcool gras (II) dans une plage de 1 : 1 à 10 : 1, en particulier dans une plage de 2 : 1 à 8 : 1, de préférence dans une plage de 3 : 1 à 6 : 1.

5. Procédé selon la revendication 1 ou la revendication 4,
où, selon la voie de synthèse (B), l'hydrolyse du groupe acyle, en particulier du groupe acétyle, a lieu en présence d'un catalyseur, de préférence d'une enzyme;
en particulier où l'enzyme est choisie parmi les synthétases (ligases), les catalases, les estérases, les lipases et leurs combinaisons; et/ou
en particulier où l'enzyme dérive de *Candida antarctica, Mucor miehei* (*Rhizomucor miehei*)*, Thermomyces lanuginosus, Candida rugosa, Aspergillus* oryzae, *Pseudomonas cepacia, Pseudomonas fluorescens, Rhizopus delemar* et *Pseudomonas* sp. ainsi que leurs combinaisons, de préférence de *Candida antarctica, Mucor miehei* (*Rhizomucor miehei*) et *Thermomyces lanuginosus*; et/ou
en particulier où l'enzyme est utilisée sous forme immobilisée, en particulier immobilisée sur un support, de préférence sur un support polymère, de préférence sur un support organique polymère, de manière particulièrement préférée avec des propriétés hydrophobes, de manière tout particulièrement préférée sur un support à base de résine poly(méth)acrylique; et/ou
en particulier où l'enzyme est utilisée en quantités, par rapport à la quantité totale du composé à hydrolyser, dans la plage de 0,001 % en poids à 20 % en poids, en particulier dans la plage de 0,01 % en poids à 15 % en poids, de préférence dans la plage de 0,1 % en poids à 15 % en poids, de préférence dans la plage de 0,5 % en poids à 10 % en poids; et/ou
en particulier où l'enzyme est recyclée après la réaction; et/ou
où, selon la voie de synthèse (B), l'hydrolyse du groupe acyle, en particulier du groupe acétyle, est réalisée à des températures dans la plage de 10 °C à 80 °C, en particulier dans la plage de 20 °C à 80 °C, de préférence dans la plage de 25 °C à 75 °C, de manière particulièrement préférée dans la plage de 45 °C à 75 °C, de manière tout particulièrement préférée dans la plage de 50 °C à 70 °C; et/ou
où, selon la voie de synthèse (B), l'hydrolyse du groupe acyle, en particulier du groupe acétyle, est réalisée à une pression dans la plage de 0,0001 bar à 10 bar, en particulier dans la plage de 0,001 bar à 5 bar, de préférence dans la plage de 0,01 bar à 2 bar, de manière particulièrement préférée dans la plage de 0,05 bar à 1 bar, de manière tout particulièrement préférée à environ 1 bar;
où, selon la voie de synthèse (B), l'hydrolyse du groupe acyle, en particulier du groupe acétyle, est réalisée en présence d'eau.

6. Procédé selon l'une quelconque des revendications précédentes,
où l'alcool gras (II) répond à la formule générale (II')
R³ - OH (Il')
où le reste R³ représente un reste alkyle en C₁₀-C₃₀ aliphatique, saturé ou mono- ou polyinsaturé, linéaire ou ramifié, de préférence un reste alkyle en C₁₀-C₂₄, en particulier où la fonction hydroxyle (fonction OH) est primaire et/ou terminale.

7. Procédé selon la revendication 6,
où le reste R³ représente un reste alkyle en C₁₀-C₂₄ aliphatique, saturé ou mono- ou polyinsaturé, linéaire, en particulier où la fonction hydroxyle (fonction OH) est primaire et/ou terminale; et/ou
où le reste R³ représente un reste 1-décanyle, un reste 1-dodécanyle (reste lauryle), un reste 1-tétradécanyle (reste myristyle), un reste 1-hexadécanyle (reste cétyle), un reste 1-heptadécanyle (reste margaryle), un reste 1-octadécanyle (reste stéaryle), un reste 1-eicosanyle (reste arachidyle), un reste 1-docosanyle (reste béhényle), un reste 1-tétracosanyle (reste ligocéryle), un reste 1-hexacosanyle (reste céryle), un reste 1-octacosanyle (reste montanyle), un reste 1-tricontanyle (reste mélissyle), un reste cis-9-hexadécén-1-yle (reste palmitoléyle), un reste cis-9-octadécén-1-yle (reste oléyle), un reste *trans*-9-octadécén-1-yle (reste élaidyle), un reste *cis*-11-octadécén-1-yle, un reste *cis,cis*-9,12-octadécén-1-yle (reste linoléyle) ou un reste 6,9,12-octadécatrién-1-yle (reste γ-linolényle), de préférence un reste *cis*-9-octadécén-1-yle (reste oléyle).

8. Procédé selon l'une quelconque des revendications précédentes,
où l'alcool gras (II) est choisi parmi les alcools gras en C₁₀-C₃₀ aliphatiques, saturés ou mono- ou polyinsaturés, linéaires ou ramifiés, en particulier les alcools gras en C₁₀-C₂₄, de préférence avec une fonction hydroxyle primaire et/ou terminale (fonction OH); et/ou
où l'alcool gras (II) est choisi parmi les alcools gras en C₁₀-C₃₀ aliphatiques, monovalents et de préférence primaires, linéaires, saturés ou mono- ou polyinsaturés, en particulier les alcools gras en C₁₀-C₂₄ aliphatiques, monovalents et de préférence primaires, linéaires, saturés ou mono- ou polyinsaturés; et/ou
où l'alcool gras (II) est choisi dans le groupe constitué par le 1-décanol, le 1-dodécanol (alcool laurylique), le 1-tétradécanol (alcool myristylique), le 1-hexadécanol (alcool cétylique), le 1-heptadécanol (alcool margarique), le 1-octadécanol (alcool stéarylique), le 1-eicosanol (alcool arachidylique), le 1-docosanol (alcool béhénylique), le 1-tétracosanol (alcool ligocérylique), le 1-hexacosanol (alcool cérylique), le 1-octacosanol (alcool montanylique), le 1-tricontanol (alcool mélisylique), le *cis*-9-hexadécén-1-ol (alcool palmitoléylique), le *cis*-9-octadécén-1-ol (alcool oléylique), le trans-9-octadécén-1-ol (alcool élaïdique), le cis-11-octadécén-1-ol, le *cis,cis*-9,12-octadécén-1-ol (alcool linoléylique), le 6,9,12-octadécatrién-1-ol (alcool γ-linolénylique), et leurs mélanges, de préférence le *cis-*9-octadécén-1-ol (alcool oléylique).

9. Ester d'alcool gras de l'acide 3-hydroxybutyrique,
où l'ester d'alcool gras de l'acide 3-hydroxybutyrique répond à la formule générale (III')
CH₃ - CH(OH) - CH₂ - C(O)OR³ (III')
où le reste R³ représente un reste 1-tétradécanyle (reste myristyle), un reste 1-hexadécanyle (reste cétyle), un reste 1-heptadécanyle (reste margaryle), un reste 1-octadécanyle (reste stéaryle), un reste 1-eicosanyle (reste arachidyle), un reste 1-docosanyle (reste béhényle), un reste 1-tétracosanyle (reste ligocéryle), un reste 1-tricontanyle (reste mélissyle), un reste cis-9-hexadécén-1-yle (reste palmitoléyle), un reste cis-9-octadécén-1-yle (reste oléyle), un reste *trans*-9-octadécén-1-yle (reste élaidyle), un reste *cis*-11-octadécén-1-yle, un reste *cis,cis*-9,12-octadécén-1-yle (reste linoléyle) ou un reste 6,9,12-octadécatrién-1-yle (reste γ-linolényle), de préférence un reste cis-9-octadécén-1-yle (reste oléyle).

10. Ester d'alcool gras de l'acide 3-hydroxybutyrique acylé (acide 3-acyloxybutyrique),
où l'ester d'alcool gras de l'acide 3-hydroxybutyrique acylé (acide 3-acyloxybutyrique) est un ester d'alcool gras de l'acide 3-hydroxybutyrique acylé (acide 3-acyloxybutyrique) répondant à la formule générale (IIIa)
CH₃ - CH(OR²) - CH₂ - C(O)OR³ (IIIa)
où dans la formule générale (IIla)
• R² représente un groupe acyle choisi parmi - C(O) - CH₃ (groupe acétyle) ou - C(O) - C₂H₅ (groupe propionyle), de préférence - C(O) - CH₃ (groupe acétyle),
• le reste R³ représente un reste 1-décanyle, un reste 1-dodécanyle (reste lauryle), un reste 1-tétradécanyle (reste myristyle), un reste 1-hexadécanyle (reste cétyle), un reste 1-heptadécanyle (reste margaryle), un reste 1-octadécanyle (reste stéaryle), un reste 1-eicosanyle (reste arachidyle), un reste 1-docosanyle (reste béhényle), un reste 1-tétracosanyle (reste ligocéryle), un reste 1-hexacosanyle (reste céryle), un reste 1-octacosanyle (reste montanyle), un reste 1-tricontanyle (reste mélissyle), un reste cis-9-hexadécén-1-yle (reste palmitoléyle), un reste cis-9-octadécén-1-yle (reste oléyle), un reste *trans*-9-octadécén-1-yle (reste élaidyle), un reste *cis*-11-octadécén-1-yle, un reste *cis,cis*-9,12-octadécén-1-yle (reste linoléyle) ou un reste 6,9,12-octadécatrién-1-yle (reste γ-linolényle), de préférence un reste cis-9-octadécén-1-yle (reste oléyle).

11. Composition pharmaceutique, en particulier médicament ou remède, comprenant un ester d'alcool gras de l'acide 3-hydroxybutyrique selon la revendication 9 ou un ester d'alcool gras de l'acide 3-hydroxybutyrique acylé (acide 3-acyloxybutyrique) selon la revendication 10.

12. Composition pharmaceutique selon la revendication 11 pour le traitement prophylactique et/ou thérapeutique et/ou pour l'utilisation dans le traitement prophylactique et/ou thérapeutique de maladies du corps humain ou animal, en particulier de maladies liées à un trouble du métabolisme énergétique, en particulier du métabolisme des corps cétoniques, comme en particulier un traumatisme crâno-cérébral, un accident vasculaire cérébral, l'hypoxie, les maladies cardiovasculaires comme l'infarctus du myocarde, le syndrome de redistribution, l'anorexie, l'épilepsie, les maladies neurodégénératives comme la démence, la maladie d'Alzheimer, la maladie de Parkinson, la sclérose en plaques et la sclérose latérale amyotrophique, les maladies du métabolisme lipidique comme le défaut du transporteur de glucose (défaut GLUT1), le VL-FAOD et les mitochondriopathies comme le défaut de la thiolase mitochondriale, la maladie de Huntington, les maladies cancéreuses comme les lymphomes à cellules **T,** les astrocytomes et les glioblastomes, le VIH, les maladies rhumatoïdes comme la polyarthrite rhumatoïde et l'arthrite rhumatoïde, les maladies du tractus gastro-intestinal comme les maladies inflammatoires chroniques de l'intestin, en particulier la rectocolite hémorragique et la maladie de Crohn, les maladies du stockage lyosomal comme les sphingolipidoses, en particulier la maladie de Niemann-Pick, le diabète sucré et les effets ou effets secondaires des chimiothérapies.

13. Ester d'alcool gras de l'acide 3-hydroxybutyrique selon la revendication 9 ou ester d'alcool gras de l'acide 3-hydroxybutyrique acylé (acide 3-acyloxybutyrique) selon la revendication 10 pour l'utilisation dans le traitement prophylactique et/ou thérapeutique de maladies du corps humain ou animal, en particulier de maladies liées à un trouble du métabolisme énergétique, en particulier du métabolisme des corps cétoniques, comme en particulier un traumatisme crâno-cérébral, un accident vasculaire cérébral, l'hypoxie, les maladies cardiovasculaires comme l'infarctus du myocarde, le syndrome de redistribution, l'anorexie, l'épilepsie, les maladies neurodégénératives comme la démence, la maladie d'Alzheimer, la maladie de Parkinson, la sclérose en plaques et la sclérose latérale amyotrophique, les maladies du métabolisme lipidique comme le défaut du transporteur de glucose (défaut GLUT1), le VL-FAOD et les mitochondriopathies comme le défaut de la thiolase mitochondriale, la maladie de Huntington, les maladies cancéreuses comme les lymphomes à cellules T, les astrocytomes et les glioblastomes, le VIH, les maladies rhumatoïdes comme la polyarthrite rhumatoïde et l'arthrite rhumatoïde, les maladies du tractus gastro-intestinal comme les maladies inflammatoires chroniques de l'intestin, en particulier la rectocolite hémorragique et la maladie de Crohn, les maladies du stockage lyosomal comme les sphingolipidoses, en particulier la maladie de Niemann-Pick, le diabète sucré et les effets ou effets secondaires des chimiothérapies.

14. Utilisation d'un ester d'alcool gras de l'acide 3-hydroxybutyrique selon la revendication 9 ou d'un ester d'alcool gras de l'acide 3-hydroxybutyrique acylé (acide 3-acyloxybutyrique) selon la revendication 10 pour la préparation d'un médicament pour le traitement prophylactique et/ou thérapeutique de maladies du corps humain ou animal, en particulier de maladies liées à un trouble du métabolisme énergétique, en particulier du métabolisme des corps cétoniques, comme en particulier un traumatisme crâno-cérébral, un accident vasculaire cérébral, l'hypoxie, les maladies cardiovasculaires comme l'infarctus du myocarde, le syndrome de redistribution, l'anorexie, l'épilepsie, les maladies neurodégénératives comme la démence, la maladie d'Alzheimer, la maladie de Parkinson, la sclérose en plaques et la sclérose latérale amyotrophique, les maladies du métabolisme lipidique comme le défaut du transporteur de glucose (défaut GLUT1), le VL-FAOD et les mitochondriopathies comme le défaut de la thiolase mitochondriale, la maladie de Huntington, les maladies cancéreuses comme les lymphomes à cellules T, les astrocytomes et les glioblastomes, le VIH, les maladies rhumatoïdes comme la polyarthrite rhumatoïde et l'arthrite rhumatoïde, les maladies du tractus gastro-intestinal comme les maladies inflammatoires chroniques de l'intestin, en particulier la rectocolite hémorragique et la maladie de Crohn, les maladies du stockage lyosomal comme les sphingolipidoses, en particulier la maladie de Niemann-Pick, le diabète sucré et les effets ou effets secondaires des chimiothérapies.

15. Produit alimentaire et/ou denrée alimentaire, comprenant un ester d'alcool gras de l'acide 3-hydroxybutyrique selon la revendication 9 ou un ester d'alcool gras de l'acide 3-hydroxybutyrique acylé (acide 3-acyloxybutyrique) selon la revendication 10.

16. Ester d'alcool gras de l'acide 3-hydroxybutyrique pour son utilisation comme médicament ou remède,
où l'ester d'alcool gras de l'acide 3-hydroxybutyrique répond à la formule générale (III')
CH₃ - CH(OH) - CH₂ - C(O)OR³ (III')
où le reste R³ représente un reste 1-tétradécanyle (reste myristyle), un reste 1-hexadécanyle (reste cétyle), un reste 1-heptadécanyle (reste margaryle), un reste 1-octadécanyle (reste stéaryle), un reste 1-eicosanyle (reste arachidyle), un reste 1-docosanyle (reste béhényle), un reste 1-tétracosanyle (reste ligocérylique), un reste 1-hexacosanyle (reste céryle), un reste 1-octacosanyle (reste montanyle), un reste 1-tricontanyle (reste mélissyle), un reste cis-9-hexadécén-1-yle (reste palmitoléyle), un reste cis-9-octadécén-1-yle (reste oléyle), un reste *trans*-9-octadécén-1-yle (reste élaidyle), un reste *cis*-11-octadécén-1-yle, un reste *cis,cis*-9,12-octadécén-1-yle (reste linoléyle) ou un reste 6,9,12-octadécatrién-1-yle (reste γ-linolényle), de préférence un reste cis-9-octadécén-1-yle (reste oléyle).

17. Ester d'alcool gras de l'acide 3-hydroxybutyrique acylé (acide 3-acyloxybutyrique) pour son utilisation comme médicament ou remède,
où l'ester d'alcool gras de l'acide 3-hydroxybutyrique acylé (acide 3-acyloxybutyrique) est un ester d'alcool gras de l'acide 3-hydroxybutyrique acylé (acide 3-acyloxybutyrique) répondant à la formule générale (IIIa)
CH₃ - CH(OR²) - CH₂ - C(O)OR³ (IIIa)
où, dans la formule générale (IIIa)
• R² représente un groupe acyle choisi parmi - C(O) - CH₃ (groupe acétyle) ou - C(O) - C₂H₅ (groupe propionyle), de préférence - C(O) - CH₃ (groupe acétyle),
• le reste R³ représente un reste 1-décanyle, un reste 1-dodécanyle (reste lauryle), un reste 1-tétradécanyle (reste myristyle), un reste 1-hexadécanyle (reste cétyle), un reste 1-heptadécanyle (reste margaryle), un reste 1-octadécanyle (reste stéaryle), un reste 1-eicosanyle (reste arachidyle), un reste 1-docosanyle (reste béhényle), un reste 1-tétracosanyle (reste ligocéryle), un reste 1-hexacosanyle (reste céryle), un reste 1-octacosanyle (reste montanyle), un reste 1-tricontanyle (reste mélissyle), un reste cis-9-hexadécén-1-yle (reste palmitoléyle), un reste cis-9-octadécén-1-yle (reste oléyle), un reste *trans*-9-octadécén-1-yle (reste élaidyle), un reste *cis*-11-octadécén-1-yle, un reste *cis,cis*-9,12-octadécén-1-yle (reste linoléyle) ou un reste 6,9,12-octadécatrién-1-yle (reste γ-linolényle), de préférence un reste cis-9-octadécén-1-yle (reste oléyle).
